# EUROPEAN PATENT SPECIFICATION

(11) **EP 4 326 194 B1**
(45) Date of publication and mention of the grant of the patent: **02.09.2026**
(21) Application number: 22722046.4
(22) Date of filing: 21.04.2022
(51) Int. Cl.: A61F 2/24

(54) **EXPANDABLE PROSTHETIC HEART VALVE WITH FLATTENED APICES**
EXPANDIERBARE HERZKLAPPENPROTHESE MIT ABGEFLACHTEN SPITZEN
VALVULE CARDIAQUE PROTHÉTIQUE EXPANSIBLE À SOMMETS APLATIS

(30) Priority: 22.04.2021 US 202163178416 P; 28.05.2021 US 202163194830 P; 13.11.2021 US 202163279096 P
(43) Date of publication of application: 28.02.2024
(73) Proprietor: Edwards Lifesciences Corporation, Irvine, CA 92614 (US)
(72) Inventor: LEVI, Tamir, S., 3079892 Caesarea (IL); GUROVICH, Nikolay, 3079892 Caesarea (IL); LAVON, Karin, 3079892 Caesarea (IL); SHERMAN, Elena, 3079892 Caesarea (IL)
(74) Representative: Eisenführ Speiser
(86) International application number: PCT/US2022/025687
(87) International publication number: WO 2022/226147

(56) References cited:
- WO-A1-2023/086548
- US-A1- 2012 197 384
- US-A1- 2013 211 499
- US-A1- 2019 053 894

## Description

### CROSS-REFERENCE TO RELATED APPLICATION

This application claims the benefit of U.S. Provisional Patent Application No. 63/178,416, filed April 22, 2021, U.S. Provisional Patent Application No. 63/194,830, filed May 28, 2021, and U.S. Provisional Patent Application No. 63/279,096, filed November 13, 2021.

### FIELD

The present disclosure relates to expandable prosthetic heart valves, including frames with apex regions having a narrowed width and reduced height.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery apparatus so that the prosthetic valve can self-expand to its functional size.

Most expandable, transcatheter heart valves comprise a radially expandable and compressible cylindrical metal frame and prosthetic leaflets mounted inside the frame. The frame can comprise a plurality of circumferentially extending rows of angled struts defining rows of open cells of the frame. The frame, at each of the inflow end and the outflow end, can comprise a plurality of apices spaced apart from one another around a circumference of the frame, each apex forming a junction between two angled struts (or strut portions) at either the inflow end or outflow end of the frame. In some cases, prosthetic heart valves have frames with angled struts that form sharp angles at the apices, thereby resulting in relatively high stress concentrations at the apices. Other prosthetic heart valves can have frames with apices having substantially vertical U-shaped portions connecting adjacent angled struts at each apex, thereby distributing the stresses across the angled struts and away from the apices. However, such apex designs can increase an overall height (in an axial direction) of the frame.

Another issue with the frame apex configurations described above includes the exposed leading or distal (e.g., inflow) apices interacting with the inflatable balloon and/or the delivery sheath through which the delivery apparatus travels en route to the implantation site. For example, in some instances, the prosthetic heart valve can be pushed toward and over the balloon, into a deployment position, upon reaching an implantation site, and cause the apices to rub against and/or abrade the balloon. In some cases, this can cause degradation to the balloon which may result in inadequate inflation at the implantation site. In other examples, during navigating through the vasculature and through a delivery sheath extending along a portion of the vasculature to the implantation site, the more pointed or sharp-angled apices at the distal end (e.g., inflow end) of the valve frame can scape against or penetrate the delivery sheath, thereby causing damage to the sheath and potentially the vasculature.

US 2019/0053894 A1 discloses an implantable prosthetic valve including a radially expandable and collapsible annular frame having an inflow end and an outflow end. A leaflet structure is positioned within the frame. An annular inner skirt is positioned around an inner surface of the frame, wherein the inner skirt includes an outflow edge portion secured to the frame and an inflow edge portion that wraps around the inflow end of the frame and extends at least partially along an outer surface of the frame, with the inflow edge portion being secured to the frame. An outer skirt is positioned around the outer surface of the frame, wherein the outer skirt comprises an outflow edge portion secured to the frame and an inwardly folded inflow edge portion that is secured to the inflow edge portion of the inner skirt.

WO 2023/086548 A1 describes a prosthetic heart valve including a plurality of leaflets attached to the frame is disclosed. The plurality of leaflets is disposed within the frame and configured to regulate the flow of blood through the frame in one direction, each leaflet comprising a cusp edge portion. The prosthetic heart valve further includes at least one suture extending along a cusp edge portion of at least a first leaflet of the plurality of leaflets, a plurality of in-and-out stitches extending through the suture and the cusp edge portion of the first leaflet, and a plurality of whip stitches that extend around selected struts of the frame and the suture to couple the cusp edge portion of the first leaflet to the frame.

Accordingly, a need exists for improved frame designs for prosthetic heart valves.

### SUMMARY

The claimed invention is defined in independent claim 1 and relates to a prosthetic heart valve. Some preferred configurations of the claimed invention are defined in dependent claims 2 to 15.

Described herein are examples of prosthetic heart valves including a radially expandable and compressible annular frame comprising a plurality of interconnected struts. The prosthetic heart valve can further include a leaflet assembly secured to the frame. In some examples, the struts of the frame can define a plurality of rows of cells arranged between an inflow end and an outflow end of the frame. The outflow end can be defined by a plurality of outflow struts and the inflow end can be defined by a plurality of inflow struts. The frame can further include a plurality of apex regions curving between two angled strut portions and forming one of the inflow struts or outflow struts with the two angled strut portions. The apex regions can be configured to be more atraumatic and have a lower height, in an axial direction. For example, in some instances, the apex regions can have a narrowed width (compared to the angled strut portions) that extends for a length, in a circumferential direction. In other instances, the apex regions (which can be alternatively referred to as apices) can have a narrowed width and a central bump that protrudes outward from thinned regions of the apex.

In one representative example, a prosthetic heart valve comprises a radially expandable and compressible annular frame comprising: a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. Each outflow strut comprises two angled strut portions interconnected by an apex region and each inflow strut comprises two angled strut portions interconnected by an apex region. Each apex region curves between a corresponding pair of two angled strut portions, where each apex region has a narrowed width and a length that extends along at least 25% of a total length of the outflow strut or inflow strut, and where the narrowed width is smaller than a width of the two angled strut portions.

In another representative example, a prosthetic heart valve comprises: a radially expandable and compressible annular frame comprising a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. Each of the plurality of outflow struts and plurality of inflow struts comprises: two angled strut portions and an apex region disposed between the two angled strut portions. The apex region comprises a curved, axially facing outer surface forming a single curve between axially facing outer surfaces of the two angled strut portions and an axially facing, inner depression that is depressed inward from axially facing inner surfaces of the two angled strut portions toward the curved outer surface of the apex region such that a width of the apex region is smaller than a width of the two angled strut portions.

In another representative example, a prosthetic heart valve comprises: a radially expandable and compressible annular frame comprising a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. Each of the plurality of outflow struts and plurality of inflow struts comprises: two angled strut portions; and an apex region disposed between the two angled strut portions, the apex region comprising an apex and two thinned strut portions extending outward from the apex in opposite directions relative to a central longitudinal axis of the apex region. A width of the two thinned strut portions is smaller than a width of the two angled strut portions and a combined length of the two thinned strut portions is at least 25% of a length of a corresponding outflow strut or inflow strut which comprises the apex region.

In another representative example, a prosthetic heart valve comprises a radially expandable and compressible annular frame comprising: a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. Each outflow strut comprises two angled strut portions interconnected by an apex region and each inflow strut comprises two angled strut portions interconnected by an apex region. Each apex region curves between a corresponding pair of two angled strut portions, wherein each apex region has a narrowed width relative to a width of the two angled strut portions. Each apex region forms an angle between the two angled strut portions that is greater than 120 degrees.

In another representative example, a prosthetic heart valve comprises a radially expandable and compressible annular frame comprising: a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. Each outflow strut comprises two angled strut portions interconnected by an apex region and each inflow strut comprises two angled strut portions interconnected by an apex region. Each apex region curves between a corresponding pair of two angled strut portions and each apex region has a narrowed width relative to a width of the two angled strut portions. Each apex region is configured to plastically deform during initial radial compression of the frame such that it becomes strain hardened and bending points of the frame are shifted to ends of the angled strut portions, away from the apex region, during subsequent radial expansion.

In another representative example, a prosthetic heart valve comprises a radially expandable and compressible annular frame comprising: a plurality of interconnected struts defining a plurality of rows of cells arranged between an inflow end and an outflow end of the frame, the plurality of rows of cells including a first row of cells disposed at the outflow end, cells of the first row of cells having a greater axial length than cells of remaining rows of cells of the plurality of rows of cells. The frame further comprises a plurality of axial struts, each axial strut defining an axial side of two adjacent cells of the first row of cells and comprising: a middle portion having a width that is greater than a width of angled struts of the plurality of interconnected struts; and an upper end portion and a lower end portion disposed on opposite ends of the middle portion and each being wider than the width of the middle portion.

In another representative example, a prosthetic heart valve comprises a radially expandable and compressible annular frame comprising: a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. Each of the plurality of outflow struts and the plurality of inflow struts comprises two angled strut portions and an apex disposed between the two angled strut portions, the apex having an axially facing inner surface comprising two inner depressions depressed into the inner surface and a central bump protruding away from and disposed between the two inner depressions. The two inner depressions form thinned regions of the apex that are smaller in width than a width of the two angled strut portions.

In another representative example, a prosthetic heart valve comprises a radially expandable and compressible annular frame comprising: a plurality of interconnected struts defining a plurality of rows of cells arranged between a first end and a second end of the frame, the plurality of interconnected struts comprising a plurality of first struts defining the first end and a plurality of second struts defining the second end, where each first strut comprises two angled strut portions interconnected by an apex. Each apex of one or more apices at the first end curves between a corresponding pair of two angled strut portions, has a narrowed width relative to a width of the two angled strut portions, and comprises a central bump protruding away from an axially facing inner surface of the apex.

In another representative example, a prosthetic heart valve comprises a radially expandable and compressible annular frame comprising: a plurality of interconnected struts defining a plurality of rows of cells arranged between an inflow end and an outflow end of the frame, the plurality of rows of cells including a first row of cells disposed at the outflow end and a plurality of axial struts. Each axial strut defines an axial side of two adjacent cells of the first row of cells and has a width that is greater than a width of angled struts of the plurality of interconnected struts. Each axial strut comprises one or more slits disposed along a length of the axial strut, the one or more slits extending through a portion of the width of the axial strut.

In another representative example, a prosthetic heart valve comprises a radially expandable and compressible annular frame comprising: a plurality of interconnected struts defining a plurality of rows of cells arranged between an inflow end and an outflow end of the frame, the plurality of rows of cells including a first row of cells disposed at the outflow end, and a plurality of axial struts, each axial strut defining an axial side of two adjacent cells of the first row of cells and having a width that is greater than a width of angled struts of the plurality of interconnected struts. Each axial strut comprises a plurality of slits spaced apart from one another along a length of the axial strut, each slit of the plurality of slits extending through a portion of the width of the axial strut.

In another representative example, a prosthetic heart valve comprises a radially expandable and compressible frame comprising: a plurality of interconnected struts defining a plurality of rows of cells arranged between a first end and a second end of the frame, the plurality of interconnected struts comprising a plurality of first struts defining the first end and a plurality of second struts defining the second end. Each first strut comprises two angled strut portions; and an apex region disposed between the two angled strut portions, the apex region curving between the two angled strut portions and having a narrowed width relative to a width of the two angled strut portions. The prosthetic heart valve further comprises a covering element wrapped around and covering the apex region of the frame.

In another representative example, a prosthetic heart valve comprises a radially expandable and compressible annular frame comprising a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. Each of the plurality of inflow struts comprises two angled strut portions; and an apex region disposed between the two angled strut portions, the apex region comprising a curved, axially facing outer surface forming a single curve between axially facing outer surfaces of the two angled strut portions and an axially facing, inner depression that is depressed inward from axially facing inner surfaces of the two angled strut portions toward the curved outer surface of the apex region such that a width of the apex region is smaller than a width of the two angled strut portions and shoulders are formed at either end of the apex region that transition from the smaller width of the apex region to the width of the two angled strut portions. The prosthetic heart valve further comprises a covering element comprising a plurality of loops wrapped around and covering at least one portion of the apex region of the frame between the shoulders of the apex region.

In another representative example, a prosthetic heart valve comprises a radially expandable and compressible annular frame comprising a plurality of interconnected struts defining a plurality of rows of cells arranged between a first end and a second end of the frame, the plurality of interconnected struts comprising a plurality of first struts defining the first end and a plurality of second struts defining the second end, wherein each of the plurality of first struts comprises an apex region; and a skirt disposed around either an inner surface or outer surface of the frame and coupled to the frame. The skirt comprises a first edge extending around a circumference of the skirt and connected to the first end of the frame; and a plurality of axially extending flaps that extend from the first edge and are spaced apart from one another, each flap of the plurality of axially extending flaps wrapped around a corresponding apex region such that the apex region is covered.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is a perspective view of a prosthetic heart valve, according to one example.
FIG. 2 is a side view of an example of a delivery apparatus configured to deliver and implant a radially expandable prosthetic heart valve at an implantation site.
FIG. 3 is a perspective view of a prosthetic heart valve, according to another example.
FIG. 4 is a side view of the prosthetic heart valve of FIG. 3 radially compressed onto and around a portion of a distal end portion of a delivery apparatus.
FIG. 5 is a side view of a frame for a prosthetic heart valve, according to another example, the frame having apices or apex regions that form a relatively large angle between angled struts of the frame at the inflow end or outflow end of the frame and that have a relatively large radius of curvature.
FIG. 6 is a magnified view of a portion of the frame of FIG. 6 showing the angle between the angled struts at the apex regions.
FIG. 7 is magnified view of a portion of a frame of a prosthetic heart valve that comprises a plurality of commissure windows that are offset away from an outflow end of the frame and disposed toward a lower portion of two adjacent cells of a rows of cells at the outflow end of the frame.
FIG. 8A is a magnified view of a portion of an exemplary frame of a prosthetic heart valve that comprises window strut portions forming a commissure window of the frame and a single aperture above the commissure window.
FIG. 8B is a magnified view of a portion of an exemplary frame of a prosthetic heart valve that comprises window strut portions forming a commissure window of the frame and two apertures above the commissure window.
FIG. 9A is a side view of a portion of a frame including outflow apex regions at an outflow end of the frame and inflow apex regions at an inflow end of the frame, the outflow and inflow apex regions having narrowed widths that extend for different lengths.
FIG. 9B is a magnified view of one of the outflow apex regions of the frame of FIG. 9A.
FIG. 9C is a magnified view of one of the inflow apex regions of the frame of FIG. 9A.
FIG. 10A is a side view of a portion of a frame including apex regions at both an outflow end and inflow end of the frame, the apex regions having a narrowed width that extends for a distance along an outflow strut or inflow strut of the frame.
FIG. 10B is a magnified view of one of the apex regions at the outflow end of the frame of FIG. 10A.
FIG. 10C is a magnified view of one of the apex regions at the inflow end of the frame of FIG. 10A.
FIG. 11 is a partial view of a frame for a prosthetic heart valve, the frame including axially extending window strut portions defining commissure windows of the frame, according to an example.
FIG. 12 shows overlapping, partial views of a frame for a prosthetic heart valve in a radially compressed configuration and a radially expanded configuration.
FIG. 13 is a perspective view of an example of a prosthetic heart valve comprising a frame and an outer skirt secured to the frame.
FIGS. 14A and 14B show an apex region of the frame of FIG. 10A, where the apex region is rotated or twisted about its axis to form a twisted outer surface.
FIG. 15 is a partial view of the frame of FIG. 10A, showing an exemplary cushioning element coupled to and covering at least a portion of an apex region of the frame.
FIG. 16A is a partial view of a frame for a prosthetic heart valve showing one apex of the frame which comprises two curved inner depressions separated from one another by a central bump.
FIG. 16B is a detail view of the single apex of FIG. 16A.
FIG. 17 is a partial view of a frame for a prosthetic heart valve showing one widened axial strut of the frame, the axial strut comprising a plurality of slits.
FIG. 18 is a partial view of a second prosthetic heart valve expanded within a previously implanted first prosthetic heart valve and a balloon bending adjacent axial struts of the first and second prosthetic heart valves away from one another to create a space for coronary access.
FIG. 19 is a partial view of the frame of FIG. 11, the frame including an axial strut with a plurality of slits configured to increase a compliance of the axial strut.
FIG. 20 is a side view of a portion of a frame of a prosthetic heart valve including apex regions and an exemplary covering element wrapped around and covering at least a portion of the apex regions.
FIG. 21 is a side view of a portion of a frame of a prosthetic heart valve including an apex region at a first end of the frame that is wrapped and covered by an exemplary covering element.
FIG. 22 is an end view of the portion of the frame of FIG. 21.
FIG. 23 is a perspective side view of a portion of a frame of a prosthetic heart valve including an apex region at a first end of the frame that is wrapped and covered by another exemplary covering element.
FIG. 24 is an end view of the portion of the frame of FIG. 23.
FIG. 25 is a side view of a portion of a prosthetic heart valve including a frame and a skirt disposed around a surface of the frame, the skirt including distal flaps configured to wrap around and cover apex regions at a first end of the frame.
FIG. 26 is a cross-sectional view of the prosthetic heart valve of FIG. 25.
FIG. 27 is a perspective view of a portion of a frame of a prosthetic heart valve, from an interior of the frame, showing an exemplary covering element extending around apex regions of the frame and through an outer skirt disposed around an outer surface of the frame such that the covering element covers at least a portion of the apex regions and secures the outer skirt to the frame.
FIG. 28A shows a first portion of an exemplary method for using a same covering element to cover apex regions of a frame and secure an outer skirt to the frame, where the covering element is used to form multiple loops around a first apex region and through the outer skirt.
FIG. 28B shows a second portion of the exemplary method for using the same covering element to cover the apex regions of the frame and secure the outer skirt to the frame, where the covering element is used to form two knots at the first apex region after forming the multiple loops around the first apex region.
FIG. 28C shows a third portion of the exemplary method for using the same covering element to cover the apex regions of the frame and secure the outer skirt to the frame, where the covering element is used to form whip stitches along an edge portion of the outer skirt, between adjacent apex regions, and then form loops around a second apex region.
FIG. 29 is a perspective view of a prosthetic heart valve, from an exterior of the prosthetic heart valve, showing an outer skirt of the prosthetic heart valve attached to an inflow end of the frame with a covering element which extends around the apex regions of the frame and through the outer skirt.
FIG. 30 is a perspective side view of a portion of a frame of a prosthetic heart valve including an axially extending strut defining a commissure window therein, where an end portion of the axially extending strut that is disposed on one side of the commissure window includes a concave region therein, at a base of an angled strut of the frame to which the axially extend strut connects.
FIG. 31 is a magnified view of a portion of a frame of a prosthetic heart valve showing another example of a concave region disposed at a transition between an outflow end portion of an axially extending strut of the frame and an angled strut of the frame.

### DETAILED DESCRIPTION

### General Considerations

For purposes of this description, certain aspects, advantages, and features of the examples of this disclosure are described herein. The described methods, systems, and apparatus should not be construed as limiting in any way. Instead, the present disclosure is directed toward all features and aspects of the various disclosed examples, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present, or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Examples of the Disclosed Technology

Described herein are examples of radially expandable and compressible prosthetic heart valves including an annular frame. The prosthetic heart valve may further include a plurality of leaflets attached to the frame. In some examples, the leaflets can be attached to the frame via commissures formed by joining pairs of adjacent ends (e.g., commissure tabs) of the leaflets.

In some examples, the frame of the prosthetic heart valve can include a plurality of rows of cells formed by interconnected struts of the frame. The plurality of rows of cells can include a first row of cells arranged at an outflow end of the frame. In some examples, the cells of the first row of cells are elongated in an axial direction relative to cells of remaining rows of cells of the frame.

The frame, at each of the inflow end and the outflow end, can comprise a plurality of apex regions spaced apart from one another around a circumference of the frame, each apex region forming a junction between two angled strut portions at either the inflow end or outflow end of the frame. For example, together, the two angles strut portions and corresponding apex region can form an outflow strut at the outflow end of the frame or an inflow strut at the inflow end of the frame. Each apex region can curve between the corresponding two angled strut portions and have a width (in a direction normal to the curve of the apex region) that is smaller than a width of the two angled strut portions.

In some examples, the apex region can have a length that is at least 25% of the length of the corresponding outflow strut or inflow strut. Further, in some examples, the apex region can have a larger radius of curvature and define a larger angle (e.g., between 120 and 140 degrees) between the two angled strut portions than more traditional apices having a more pointed or U-shape. This can result in the apex regions having a relatively small overall height, defined in an axial direction (e.g., equal to the width of the apex region which is smaller than the width of the angled strut portions).

In some examples, the apex region can comprise two curved inner depressions separated from one another by a micro-sized bump.

In this way, the relatively small height the apex regions can allow for cells at the outflow end of the frame to have a longer axial length, thereby resulting in more open space at an outflow end of the cells for blood flow and coronary access. Further, the configuration of the apex regions described above can provide more atraumatic apex regions at the outflow end and inflow end of the frame, thereby reducing interaction between the apex regions and a balloon of a delivery apparatus and/or a delivery sheath.

Further, in some examples, a cushioning or covering element can cover and/or be wrapped around at least a portion of the apices or apex region at an end of the frame, thereby providing even more atraumatic apex regions or apices and potentially reducing push forces through a delivery sheath during navigation of the radially compressed prosthetic heart valve to an implantation site via a delivery apparatus.

Prosthetic valves disclosed herein can be radially compressible and expandable between a radially compressed state and a radially expanded state. Thus, the prosthetic valves can be crimped on or retained by an implant delivery apparatus in the radially compressed state during delivery, and then expanded to the radially expanded state once the prosthetic valve reaches the implantation site. It is understood that the prosthetic valves disclosed herein may be used with a variety of implant delivery apparatuses and can be implanted via various delivery procedures, examples of which will be discussed in more detail later.

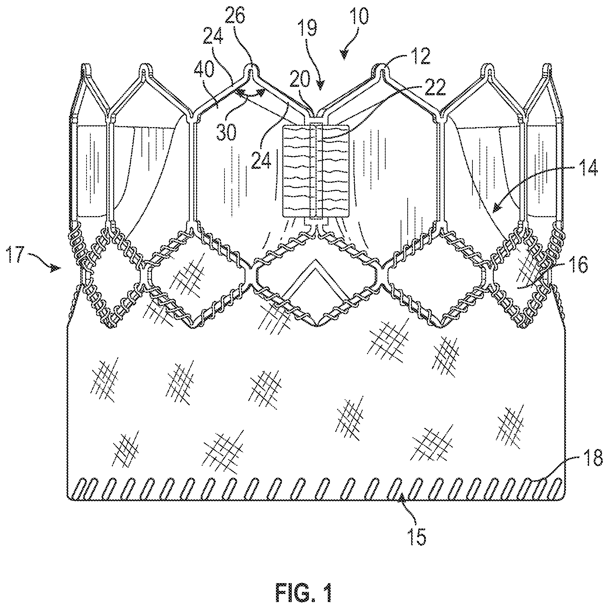

FIG. 1 shows a prosthetic heart valve 10, according to one example. Any of the prosthetic valves disclosed herein are adapted to be implanted in the native aortic annulus, although in other examples they can be adapted to be implanted in the other native annuluses of the heart (e.g., the pulmonary, mitral, and tricuspid valves). The disclosed prosthetic valves also can be implanted within vessels communicating with the heart, including a pulmonary artery (for replacing the function of a diseased pulmonary valve, or the superior vena cava or the inferior vena cava (for replacing the function of a diseased tricuspid valve) or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756. In another example, the disclosed prosthetic valves can be implanted within a docking device implanted within or at the native mitral valve, such as disclosed in PCT Publication No. WO2020/247907. In another example, the disclosed prosthetic valves can be implanted within a docking device implanted within the superior or inferior vena cava for replacing the function of a diseased tricuspid valve, such as disclosed in U.S. Publication No. 2019/0000615.

The prosthetic valve 10 can have four main components: a stent or frame 12, a valvular structure 14, an inner skirt 16, and a perivalvular outer sealing member or outer skirt 18. The prosthetic valve 10 can have an inflow end portion 15, an intermediate portion 17, and an outflow end portion 19. The inner skirt 16 can be arranged on and/or coupled to an inner surface of the frame 12 while the outer skirt 18 can be arranged on and/or coupled to an outer surface of the frame 12.

The valvular structure 14 can comprise three leaflets 40, collectively forming a leaflet structure, which can be arranged to collapse in a tricuspid arrangement, although in other examples there can be greater or fewer number of leaflets (e.g., one or more leaflets 40). The leaflets 40 can be secured to one another at their adjacent sides to form commissures 22 of the leaflet structure 14. The lower edge of valvular structure 14 can have an undulating, curved scalloped shape and can be secured to the inner skirt 16 by sutures (not shown). In some examples, the leaflets 40 can be formed of pericardial tissue (e.g., bovine pericardial tissue), biocompatible synthetic materials, or various other suitable natural or synthetic materials as known in the art and described in U.S. Patent No. 6,730,118.

The frame 12 can be radially compressible (collapsible) and expandable (e.g., expanded configuration shown in FIG. 1) and comprise a plurality of interconnected struts 24. A plurality of apices 26 that are spaced circumferentially apart are formed at the inflow end portion 15 and the outflow end portion 19 of the frame 12 (only the apices 26 at the outflow end portion 19 are visible in FIG. 1). Each apex 26 is formed at a junction between two angled struts 24 at either the inflow end portion 15 or the outflow end portion 19. FIG. 1 depicts a known frame design with apices 26 that form a U-shaped bend between the two angled struts 24. In some examples, an angle 30 between the two angled struts 24, connected at the apex 26, can be in a range of 90 to 120 degrees.

The frame 12 can be formed with a plurality of circumferentially spaced slots, or commissure windows 20 that are adapted to mount the commissures 22 of the valvular structure 14 to the frame. The frame 12 can be made of any of various suitable plastically-expandable materials (e.g., stainless steel, etc.) or self-expanding materials (e.g., Nitinol). When constructed of a plastically-expandable material, the frame 12 (and thus the prosthetic valve 10) can be crimped to a radially collapsed configuration on a delivery catheter or apparatus and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the sheath, which allows the prosthetic valve to expand to its functional size.

Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular examples, frame 12 is made of a nickel-cobalt-chromium-molybdenum alloy, such as MP35N^{®} alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight. Additional details regarding the prosthetic valve 10 and its various components are described in WIPO Patent Application Publication No. WO 2018/222799.

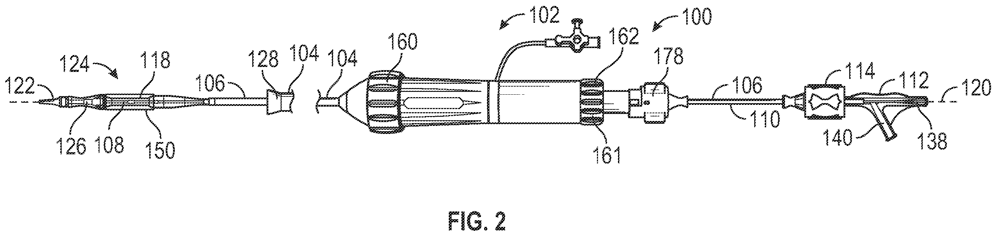

FIG. 2 shows a delivery apparatus 100, according to an example, that can be used to implant an expandable prosthetic heart valve (e.g., prosthetic heart valve 10 of FIG. 1 or any of the other prosthetic heart valves described herein). In some examples, the delivery apparatus 100 is specifically adapted for use in introducing a prosthetic valve into a heart.

The delivery apparatus 100 in the illustrated example of FIG. 2 is a balloon catheter comprising a handle 102 and a steerable, outer shaft 104 extending distally from the handle 102. The delivery apparatus 100 can further comprise an intermediate shaft 106 (which also may be referred to as a balloon shaft) that extends proximally from the handle 102 and distally from the handle 102, the portion extending distally from the handle 102 also extending coaxially through the outer shaft 104. Additionally, the delivery apparatus 100 can further comprise an inner shaft 108 extending distally from the handle 102 coaxially through the intermediate shaft 106 and the outer shaft 104 and proximally from the handle 102 coaxially through the intermediate shaft 106.

The outer shaft 104 and the intermediate shaft 106 can be configured to translate (e.g., move) longitudinally, along a central longitudinal axis 120 of the delivery apparatus 100, relative to one another to facilitate delivery and positioning of a prosthetic valve at an implantation site in a patient's body.

The intermediate shaft 106 can include a proximal end portion 110 that extends proximally from a proximal end of the handle 102, to an adaptor 112. A rotatable knob 114 can be mounted on the proximal end portion 110 and can be configured to rotate the intermediate shaft 106 around the central longitudinal axis 120 and relative to the outer shaft 104.

The adaptor 112 can include a first port 138 configured to receive a guidewire therethrough and a second port 140 configured to receive fluid (e.g., inflation fluid) from a fluid source. The second port 140 can be fluidly coupled to an inner lumen of the intermediate shaft 106.

The intermediate shaft 106 can further include a distal end portion that extends distally beyond a distal end of the outer shaft 104 when a distal end of the outer shaft 104 is positioned away from an inflatable balloon 118 of the delivery apparatus 100. A distal end portion of the inner shaft 108 can extend distally beyond the distal end portion of the intermediate shaft 106.

The balloon 118 can be coupled to the distal end portion of the intermediate shaft 106.

In some examples, a distal end of the balloon 118 can be coupled to a distal end of the delivery apparatus 100, such as to a nose cone 122 (as shown in FIGS. 2), or to an alternate component at the distal end of the delivery apparatus 100 (e.g., a distal shoulder). An intermediate portion of the balloon 118 can overlay a valve mounting portion 124 of a distal end portion of the delivery apparatus 100 and a distal end portion of the balloon 118 can overly a distal shoulder 126 of the delivery apparatus 100. The valve mounting portion 124 and the intermediate portion of the balloon 118 can be configured to receive a prosthetic heart valve in a radially compressed state. For example, as shown schematically in FIG. 2, a prosthetic heart valve 150 (which can be one of the prosthetic valves described herein) can be mounted around the balloon 118, at the valve mounting portion 124 of the delivery apparatus 100.

The balloon shoulder assembly, including the distal shoulder 126, is configured to maintain the prosthetic heart valve 150 (or other medical device) at a fixed position on the balloon 118 during delivery through the patient's vasculature.

The outer shaft 104 can include a distal tip portion 128 mounted on its distal end. The outer shaft 104 and the intermediate shaft 106 can be translated axially relative to one another to position the distal tip portion 128 adjacent to a proximal end of the valve mounting portion 124, when the prosthetic valve 150 is mounted in the radially compressed state on the valve mounting portion 124 (as shown in FIG. 2) and during delivery of the prosthetic valve to the target implantation site. As such, the distal tip portion 128 can be configured to resist movement of the prosthetic valve 150 relative to the balloon 118 proximally, in the axial direction, relative to the balloon 118, when the distal tip portion 128 is arranged adjacent to a proximal side of the valve mounting portion 124.

An annular space can be defined between an outer surface of the inner shaft 108 and an inner surface of the intermediate shaft 106 and can be configured to receive fluid from a fluid source via the second port 140 of the adaptor 112. The annular space can be fluidly coupled to a fluid passageway formed between the outer surface of the distal end portion of the inner shaft 108 and an inner surface of the balloon 118. As such, fluid from the fluid source can flow to the fluid passageway from the annular space to inflate the balloon 118 and radially expand and deploy the prosthetic valve 150.

An inner lumen of the inner shaft can be configured to receive a guidewire therethrough, for navigating the distal end portion of the delivery apparatus 100 to the target implantation site.

The handle 102 can include a steering mechanism configured to adjust the curvature of the distal end portion of the delivery apparatus 100. In the illustrated example, for example, the handle 102 includes an adjustment member, such as the illustrated rotatable knob 160, which in turn is operatively coupled to the proximal end portion of a pull wire. The pull wire can extend distally from the handle 102 through the outer shaft 104 and has a distal end portion affixed to the outer shaft 104 at or near the distal end of the outer shaft 104. Rotating the knob 160 can increase or decrease the tension in the pull wire, thereby adjusting the curvature of the distal end portion of the delivery apparatus 100. Further details on steering or flex mechanisms for the delivery apparatus can be found in U.S. Patent No. 9,339,384.

The handle 102 can further include an adjustment mechanism 161 including an adjustment member, such as the illustrated rotatable knob 162, and an associated locking mechanism including another adjustment member, configured as a rotatable knob 178. The adjustment mechanism 161 is configured to adjust the axial position of the intermediate shaft 106 relative to the outer shaft 104 (e.g., for fine positioning at the implantation site). Further details on the delivery apparatus 100 can be found in U.S. Provisional Application Nos. 63/069,567 and 63/138,890.

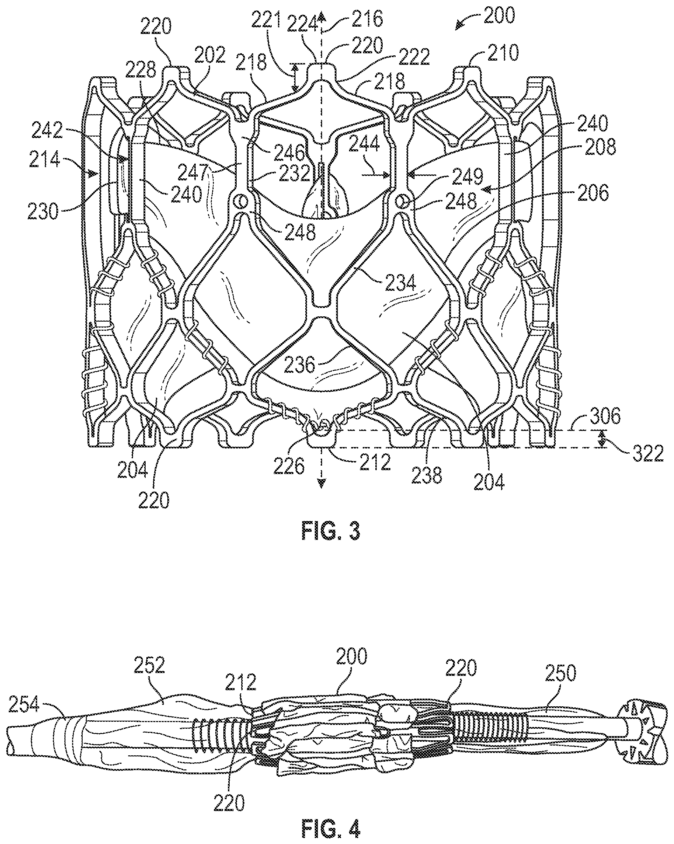

FIG. 3 shows an example of a prosthetic heart valve 200 comprising a radially expandable and compressible annular frame 202 and a plurality of leaflets 204 secured to the frame. Each leaflet 204 can comprise opposing commissure tabs disposed on opposite sides of the leaflet 204 and a cusp edge portion extending between the opposing commissure tabs.

The frame 202 can be made of any of various suitable plastically-expandable materials (e.g., stainless steel, etc.) or self-expanding materials (e.g., nickel titanium alloy (NiTi), such as nitinol), as known in the art. In some examples, the frame 202 comprises a plastically-expandable material, such as those described above with reference to the prosthetic heart valve 10 of FIG. 1.

The frame 202 can comprise a plurality of interconnected struts 206 which form multiple rows of open cells 208 between an outflow end 210 and an inflow end 212 of the frame 202. In some examples, as shown in FIG. 3, the frame 202 can comprise three rows of cells 208 with a first (e.g., upper in FIG. 3) row of cells 214, disposed at the outflow end 210, having cells 208 that are elongated in an axial direction (relative to a central longitudinal axis 216 of the frame 202), as compared to cells 208 in the remaining rows of cells. For example, the cells 208 of the first row of cells 214 can have a longer axial length, defined in a direction of a central longitudinal axis 216 of the frame 202, than cells 208 in the remaining rows of cells (e.g., cells in the row of cells at the inflow end 212).

In some examples, as shown in FIG. 3, each row of cells 208 comprises nine cells. Thus, in such examples, the frame 202 can be referred to as a nine-cell frame.

In other examples, the frame 202 can comprise more than three rows of cells (e.g., four or five) and/or more or less than nine cells per row. In some examples, the cells 208 in the first row of cells 214 may not be elongated compared to cells 208 in remaining rows of cells of the frame 202.

The interconnected struts 206 can include a plurality of angled struts 218, 234, 236, and 238 arranged in a plurality of rows of circumferentially extending rows of angled struts, with the rows being arrayed along the length of the frame between the outflow end 210 and the inflow end 212 of the frame 202. For examples, the frame 202 can comprise a first row of angled struts 238 arranged end-to-end and extending circumferentially at the inflow end 212 of the frame; a second row of circumferentially extending, angled struts 236; a third row of circumferentially extending, angled struts 234; and a fourth row of circumferentially extending, angled struts 218 at the outflow end 210 of the frame 12. The fourth row of angled struts 218 can be connected to the third row of angled struts 234 by a plurality of axially extending window strut portions 240 and a plurality of axial (e.g., axially extending) struts 232. The axially extending window strut portions 240 define commissure windows (e.g., open windows) 242 that are spaced apart from one another around the frame 202, in a circumferential direction, and which are adapted to receive a pair of commissure tabs of a pair of adjacent leaflets 204 arranged into a commissure 230.

One or more (e.g., two, as shown in FIG. 3) axial struts 232 can be positioned between, in the circumferential direction, two commissure windows 242 formed by the window strut portions 240. Since the frame 202 can include fewer cells per row (e.g., nine) and fewer axial struts 232 between each commissure window 242, as compared to other prosthetic heart valves, such as the prosthetic heart valve 10 of FIG. 1, each cell 208 can have an increased width (in the circumferential direction), thereby providing a larger opening for blood flow and/or coronary access, as described herein.

Each axial strut 232 and each window strut portion 240 extends from a location defined by the convergence of the lower ends (e.g., ends arranged inward of and farthest away from the outflow end 210) of two angled struts 218 (which can also be referred to as an upper strut junction or upper elongated strut junction) to another location defined by the convergence of the upper ends (e.g., ends arranged closer to the outflow end 210) of two angled struts 234 (which can also be referred to as a lower strut junction or lower elongate strut junction). Each axial strut 232 and each window strut portion 240 forms an axial side of two adjacent cells of the first row of cells 214.

In some examples, as shown in FIG. 3, each axial strut 232 can have a width 244 that is larger than a width of the angled struts 218, 234, 236, and/or 238. As used herein, a "width" of a strut is measured between opposing locations on opposing surfaces of a strut that extend between the radially facing inner and outer surfaces of the strut (relative to the central longitudinal axis 216 of the frame 200). A "thickness" of a strut is measured between opposing locations on the radially facing inner and outer surfaces of a strut and is perpendicular to the width of the strut. In some examples, the width 244 of the axial struts 232 is 50-200%, 75-150%, or at least 100% larger than (e.g., double) the width of the angled struts of the frame 202. For example, if the angled struts 218, 234, 236, and 238 are approximately 0.3 mm, then the width 244 of the axial struts 232 can be in a range of 0.45 mm - 0.9 mm, 0.5 mm - 0.75 mm, or at least 0.6 mm. In some examples, the width 244 of the axial struts 232 can be in range of 0.5 mm - 1.0 mm.

For example, in known prosthetic heart valves (such as the valve 10 shown in FIG. 1) the axial struts have a same width as the other struts (e.g., angled struts) of the frame. However, when implanted and during use, when the leaflets of the prosthetic heart valve are pressed against the frame during the systolic phase, the leaflets may bend around the struts radially outwardly through the cell openings due to the relatively narrow width of the axial struts. This phenomenon can reduce long-term durability of the leaflets, especially when the upper (e.g., outflow) edges of the leaflets are pushing against the axial struts and are bent over, as described above.

Thus, by providing the axial struts 232 with the width 244 that is greater than the width of other, angled struts of the frame 202, a larger contact area is provided for when the leaflets 204 contact the wider axial struts 232 during systole, thereby distributing the stress and reducing the extent to which the leaflets 204 may fold over the axial struts 232, radially outward through the cells 208. As a result, a long-term durability of the leaflets 204 can be increased.

In some cases, the free edges at the outflow end 228 of the leaflets 204 may press against the axial struts 232 at their outflow (e.g., upper) end portions 246. However, these outflow end portions 246 can be even wider than the width 244 (as shown in FIG. 3), thereby providing an even larger area of contact and support for the leaflets 204.

As introduced above, since the frame 202 can have fewer cells in the circumferential direction (e.g., nine in FIG. 3) compared to the prosthetic valve of FIG. 1, each cell 208 can have an increased width (measured in the circumferential direction). This increased width of the cells 208 of the first row of cells 214 can enable the wider axial struts 232 to be incorporated into the frame 202, without sacrificing open space for blood flow and/or coronary access.

Commissure tabs of adjacent leaflets 204 can be secured together to form commissures 230. Each commissure 230 of the prosthetic heart valve 200 comprises two commissure tabs paired together, one from each of two adjacent leaflets 204, and extending through a commissure window 242 of the frame 202. Each commissure 230 can be secured to the window strut portions 240 forming the commissure window 242.

The cusp edge portion (e.g., scallop edge) of each leaflet 204 can be secured to the frame via one or more fasteners (e.g., sutures). In some examples, as shown in FIG. 3, the cusp edge portion of each leaflet 204 can be secured directly to the struts of the frame 202 (e.g., angled struts 234, 236, and 238). For example, the cusp edge portions of the leaflets 204 can be sutured to the angled struts 234, 236, 238 that generally follow the contour of the cusp edge portions of the leaflets.

In some examples, the cusp edge portion of the leaflets 204 can be secured to an inner skirt and the inner skirt can then be secured directly to the frame 202.

Further, in some examples, an outer skirt can be connected to an outer surface of the frame 202 (e.g., similar to the outer skirt 18 of the valve 10 of FIG. 1).

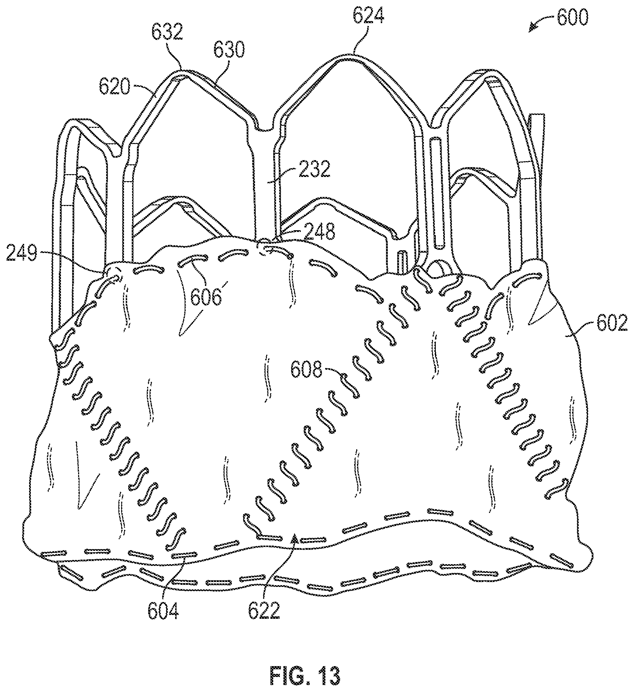

As shown in FIG. 3, in some examples, one or more of or each of the axial struts 232 can comprise an inflow end portion (e.g., inflow end portion that is closer to the inflow end than the outflow end portion 246) 248 that is widened relative to a middle portion 247 of the axial strut 232 (which can be defined by the width 244), similar to the outflow end portion 246 (as described above). In some examples, the inflow end portion 248 of the axial strut 232 can comprise an aperture 249. The apertures 249 can be configured to receive fasteners (e.g., sutures) for attaching soft components of the prosthetic heart valve 200 to the frame 202. For example, in some instances, an outer skirt can be positioned around an outer surface of the frame 202 and secured to the apertures 249 (e.g., as shown in FIG. 13 and described further below).

The frame 202 can further comprise a plurality of apices 220 formed at the inflow end 212 and the outflow end 210, each apex 220 forming a junction between two angled struts 218 at the inflow end 212 or outflow end 210. As such, the apices 220 are spaced apart from one another, in a circumferential direction at the inflow end 212 and the outflow end 210. As shown in FIG. 3, each apex 220 can have side portions 222 that curve or bend axially outward from the angled strut 218 to which it is connected and an end portion 224 that extends between the two side portions 222 of the apex 220. The side portions 222 can extend in a direction that is parallel to the central longitudinal axis 216. The end portion 224 can be relatively flat and include a surface that is disposed normal to the central longitudinal axis 216. Each apex 220 can have two bends at its end portion 224 and two bends at the side portions 222 (e.g., one at the junction between each side portion 222 and angled strut 218). In this way, the apices 220 can be U-shaped, similar to the apices 26 of the valve of FIG. 1.

While apices 220 of such a shape can distribute stresses at the apices 220 across the angled struts 218 to which they are connected, such a shape results in adding the height (in the axial direction) 221 of the apices 220 at the inflow end 212 and the outflow end 210 to the overall height of the prosthetic heart valve 200. As a result, the inflow end 226 of the leaflets 204 are spaced away from the inflow end 212 of the frame 202. This, in turn, can cause the outflow end 228 of the leaflets 204 to be disposed closer to the outflow end 210 of the frame, thereby leaving less open space in the cells 208 of the upper row of cells 214 between the outflow edges of the leaflets and the upper row of struts 218. In some examples, this arrangement can result in the leaflets 204, including commissures 230 of adjacent commissure tabs of the leaflets 204, to at least partially block blood flow into the coronary ostia. Further, coronary re-access devices can be inhibited from passing through such small spaces.

FIG. 4 shows the prosthetic heart valve 200 radially compressed onto and around a portion of a distal end portion of a delivery apparatus 250 (which may be the same or similar to the delivery apparatus 100 of FIG. 2). As shown in FIG. 4, the prosthetic heart valve 200 is radially compressed around a portion of an inflatable balloon 252 of the delivery apparatus 250 (the inflatable balloon 252 may be similar to the balloon 118 of FIG. 2). In some examples, as shown in FIG. 4, the prosthetic heart valve 200 can be crimped onto the distal end portion of the delivery apparatus 250 with the inflow end 212 facing a nose cone 254 of the delivery apparatus 250. In other examples, the prosthetic heart valve 200 can be crimped onto the distal end portion of the delivery apparatus 250 with the outflow end 210 facing a nose cone 254.

In such a configuration, at least the apices 220 at the inflow end 212 are exposed which can result in abrasion of the apices 220 against or penetration of the apices 220 into an inner wall of a delivery sheath through which the delivery apparatus 250 travels en route to the implantation site (e.g., during delivery of the prosthetic heart valve 200 to the target implantation site). For example, during an implantation procedure when the prosthetic heart valve 200 is radially compressed (e.g., crimped) around the delivery apparatus 250, the prosthetic heart valve 200 can be pushed through an inner lumen of the delivery sheath and the apices 220 can contact, puncture, or tear the walls of the delivery sheath.

In some examples, the exposed apices 220 can interact with the balloon 252. For example, if the prosthetic heart valve 200 is crimped off an inflatable portion of the balloon 252, upon reaching the implantation site when the prosthetic heart valve 200 is pushed over the inflatable portion of the balloon 252, the inflow apices 220 can interact with the balloon 252 (e.g., scrape against or resist movement of the valve over the balloon).

To address the above-described issues with such U-shaped or more pointed apices that add height to the inflow and outflow ends of the prosthetic heart valve, a frame of a prosthetic heart valve can instead have apices or apex regions that form a larger angle between the angled struts (or angled strut portions) at the inflow end or outflow end of the frame and that have a larger radius of curvature, thereby creating a more curved or flattened shape (e.g., less sharp angled, such as the U-shaped design shown in FIGS. 1 and 3) that can be more atraumatic to the balloon of the delivery apparatus and the delivery sheath. Additionally, as explained further below, such apex regions can create a smaller axial height at the inflow and outflow ends of the prosthetic heart valve, thereby allowing the inflow ends (e.g., the inflow end of the cusp edge portion) of the leaflets to be positioned closer to the inflow end of the frame, and thus, creating more open space at the outflow end of the prosthetic heart valve for increased blood flow and coronary access.

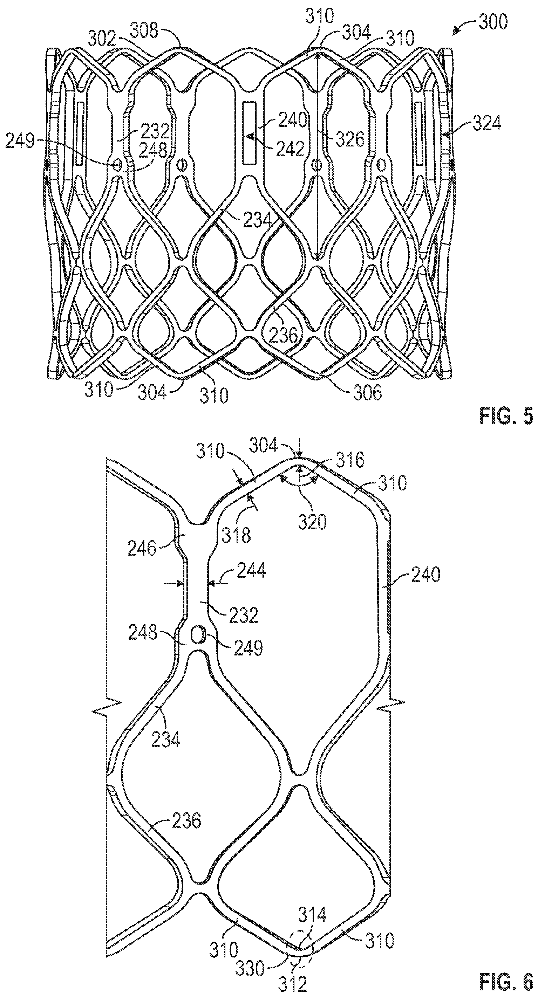

FIG. 5 shows an exemplary frame 300 for a prosthetic heart valve comprising interconnected struts 302 forming apices (or apex regions) 304 at an inflow end 306 and outflow end 308 of the frame 300. As shown in FIG. 5 and the magnified view of a portion of the frame 300 of FIG. 6, each apex 304 is disposed between and forms a transition between two angled struts 310 (similar to angled struts 218 and 238 of frame 202 of FIG. 3) at the inflow end 306 or outflow end 308 of the frame 300.

Each apex 304 can have a more flattened (e.g., less pointed) shape as compared to the apices 220 of FIG. 3. For example, each apex 304 can include a curved or relatively flat outer surface 312 and an arcuate or curved inner depression 314 disposed opposite the outer surface 312 (FIG. 6). The inner depression 314 forms a thinned region at the apex 304, having a width 316 that is smaller than a width 318 of the angled struts 310 (FIG. 6). This thinned region of the apex 304 can spread stresses experienced by the frame 300 away from the apex and across both the angled struts 310 extending from either side of the apex 304.

Each apex 304 of the frame 300 (FIGS. 5 and 6) can have a reduced height compared to each apex 220 of the frame 202 (FIG. 3). For example, FIG. 3 shows a height difference 322 between the inflow end 212 of the frame 202 (at apex 220) and where the inflow end 306 of the frame 300 would be positioned in comparison, due to the more flattened apices 304 of the frame 300. As a result, the inflow end 226 of the leaflets 204 can be positioned closer to the inflow end 306 of the frame 300 than in the frame 202. In some examples, for a same overall valve height for the frame 300 and frame 202, this allows the cells of the frame 300, particularly the first row of cells 324 disposed at or adjacent to the outflow end 308, to be lengthened in the axial direction. For example, in some instances, an axial height 326 of the first row of cells 324 (FIG. 5) can be increased by about two times the height 221 of the more U-shaped and axially-oriented apices 220 of the frame 202 (FIG. 3) from the axial height of the first row of cells 214 of the frame 202. These "higher" or longer first row of cells 324 of the frame 300 can have a larger portion that remains exposed above the outflow ends of the leaflets, thereby creating more open space for blood flow at the outflow end of the frame 300 and reducing the risk of sinus sequestration.

In some examples, as shown in FIG. 6, an angle (e.g., apex angle) 320 between the two angled struts 310 connected at the apex 304 can be greater than 120 degrees. In some examples, the angle 320 can be in a range of 120 (not inclusive) to 140 degrees (e.g., such that the angle 320 is greater than 120 degrees). In some examples, the angle 320 can be in a range of 135-140 degrees, 138-140 degrees, or 139-140 degrees. In some examples, the angle 320 can be approximately 140 degrees (e.g., ±1 degree).

In some examples, a balloon expandable valve comprising the frame 300 can be radially expanded and deployed at a target implantation site by inflating a balloon of a delivery apparatus around which the prosthetic heart valve is mounted (e.g., such as the delivery apparatus of FIG. 2). Following such deployment, due to a small amount of inherent resiliency of the metal forming the frame 300, the frame tends to recoil radially inward (toward a central longitudinal axis of the frame) to an expanded diameter that is slightly smaller than the diameter defined by the inflated balloon (of the delivery apparatus), once the balloon is deflated and no longer exerts a radially outward force on the frame 300. It may be desirable for such radial recoil to be in a range of less than five percent of the diameter of the prosthetic heart valve when radially expanded over the inflated balloon. Reducing the radial recoil from the diameter of the valve on the inflated balloon to the diameter of the valve after deflating the balloon can provide better predictability of the final expanded diameter of the valve.

The angle 320 at the apex 304 can influence the degree of recoil of the frame 300 after radially expanding the frame of the prosthetic heart valve via inflating and deflating the balloon. For example, larger apex angles (e.g., greater than 120 and up to 140 degrees) can result in a smaller extent of a radial recoil, as compared to smaller apex angles (e.g., between 90 and 120 degrees).

Apices 304 that create the angle 320 between the angled struts 310 in the range described above (e.g., greater than 120 degrees and up to 140 degrees) can result in a radial recoil at the inflow end 306 and outflow end 308 in the range of 0.3-0.4 mm for a prosthetic valve having an expanded working diameter of 20 mm, which is less than half the radial recoil that can be experienced at the mid-portion of the valve (e.g., in a range of 0.95 - 0.97 mm). The radial recoil at the inflow end 306 and outflow end 308 of the frame 202 can also be significantly lower than the radial recoil of a valve with more conventional apices and apex angles of 120 degrees or less (e.g., such as the valve 10 of FIG. 1), which can be in the range of 0.7-0.8 mm.

Advantageously, the configuration of the apices 304, as described above, allow for enlargement of the apex angle 320, beyond the angle of more traditional U-shaped apex angles, thereby reducing the extent of radial recoil of the frame 300. Further, the increased angle and shape of the apices 304 (e.g., reduced height and curved or arcuate shape, as described above) provide for apices 304 that are more atraumatic (compared to more pointed and/or U-shaped apices, such as the apices shown in FIGS. 1 and 3). As a result, a risk of the apices 304 interacting with and/or degrading the delivery sheath and/or inflatable balloon of the delivery apparatus, as well as the native anatomy, can be reduced.

Other than the configuration of the apices 304, the frame 300 can be similar to the frame 202 of FIG. 3. For example, the frame 300 can comprise a first row of cells 324 that are defined by the angled struts 310 at the outflow end 308, the angled struts 234, and window strut portions 240 and axial struts 232.

In some examples, as shown in FIGS. 5 and 6, one or more or each of the axial struts 232 can have the increased width 244, as described above with reference to FIG. 3. Further, one or more of or each of the axial struts 232 can comprise the aperture 249 in the inflow end portion 248.

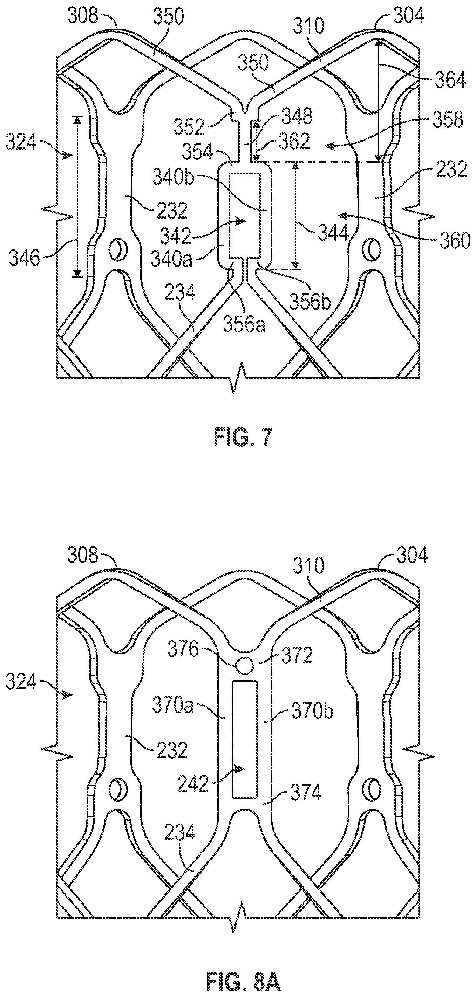

In some examples, instead of being centered along the window strut portions 240 and between two adjacent cells of the upper row of cells 324 (e.g., as shown in FIG. 5), a commissure window 342 can be positioned along a lower portion of axial struts forming the axial sides of the two adjacent cells of the first row of cells 324, as shown in FIG. 7. Said another way, the commissure window 342 can be spaced away from the outflow end 308 of the frame 300 and toward a lower portion of the two adjacent cells of the first row of cells 324.

For example, FIG. 7 shows a portion of an example of the frame 300 including the commissure window 342 which is lowered within the first row of cells 324 (compared to the commissure windows 242 shown in FIG. 5). The commissure window 342 is formed and defined between a first axially extending window strut portion 340a and a second axially extending window strut portion 340b. As shown in FIG. 7, a length 344 of the axially extending window strut portions 340a and 340b is shorter than a length 346 of the axial struts 232. The axially extending window strut portions 340a and 340b are integrally formed with the frame 300, each extending from an upper (e.g., proximal) axial strut 348. Together, the axially extending window strut portions 340a and 340b and the upper axial strut 348 can form an axially extending frame portion defining an axial side of each of two adjacent elongated cells 350 of the upper row of cells 324.

In some examples, a width of the upper axial strut 348, defined in the circumferential direction and perpendicular to the central longitudinal axis of the frame, can be the same as a width of the axially extending window strut portions 340a and 340b and/or additional struts of the frame (e.g., angled struts 310).

In some examples, the width of the upper axial strut 348 can be larger (e.g., thicker) than the width of the axially extending window struts 340a and 340b and/or additional struts of the frame (e.g., angled struts 310).

In some examples, the upper axial strut 348 can include additional geometrical features, such as one or more holes or notches along its length.

The upper axial strut 348 is arranged between an upper (e.g., proximal) elongated strut junction 352 (e.g., the junction between two angled struts 310 and the upper axial strut 348) and an upper edge 354 of the commissure window 342. The upper edge 354 is arranged substantially perpendicular to the upper axial strut 348 and laterally (e.g., circumferentially) offsets the axially extending window strut portions 340a and 340b from each other. Said another way, the upper edge 354 can extend between, in the circumferential (or lateral) direction, upper ends of each of the axially extending window strut portions 340a and 340b. As used herein, "upper" ends, struts, or edges can refer to ends, struts, or edges of components that are disposed closer to a proximal or outflow end 308 of the frame 300 and "lower" ends, struts, or edges can refer to ends, struts, or edges of components that are disposed further away from the outflow end 308 and toward the inflow end 306.

As shown in FIG. 7, in some examples, what would be a lower elongated strut junction connecting the lower ends of strut portions 340a, 340b is replaced with an open, lower end of the commissure window 342. For example, in some instances, each axially extending window strut portion 340a and 340b includes a lower clamping portion 356a and 356b, respectively, formed by a lower, bent (e.g., angled) portion of the respective axially extending window strut portions 340a and 340b. As shown in FIG. 7, the bends of the lower clamping portions 356a and 356b are angled toward each other. Thus, as shown in FIG. 7, together, the lower clamping portions 356a and 356b can form a neck region at the lower end of the open commissure window 342.

In some examples, commissure tabs of a commissure can extend through the open commissure window 342 to form a commissure assembly (e.g., commissure assembled to the frame 300). For example, in some instances, commissure tabs two adjacent leaflets can be inserted into the commissure window 342, through an opening defined between the lower clamping portions 356a and 356b.

In some examples, the axially extending window struts 340a and 340b and their respective lower clamping portions 356a and 356b may be flexed or resiliently bent sideways (e.g., laterally outward and away from one another) during leaflet insertion.

In some examples, a suture can be wrapped or looped around the lower clamping portions 356a and 356b (e.g., wrapped or looped around outer bends in each of the lower clamping portions), once the commissure tabs are positioned within the commissure window 342, so as to clamp the lower ends of the axially extending window struts 340a and 340b to each other, and to prevent the commissure tabs from sliding axially out of the commissure window 34. In alternate examples, alternative fasteners or clamping means (e.g., bands, strings, ties, or the like) can be used to clamp the lower clamping portions 356a and 356b together.

In other examples, the lower end of each commissure window 342 can be closed and formed by a lower elongated strut junction (e.g., similar to junction 352).

Further, in some examples, the axial position (along the central longitudinal axis of the frame 300) of the upper edge 354, or the axial distance between the upper edge 354 and the outflow end 308 of the frame, can be selected such that the entirety of the leaflets or at least a majority of the leaflets are maintained within a lower portion 358 of the cells of the first row of cells 324 and an upper portion 360 of the cells of the upper row of cells 324 remain substantially unblocked by the leaflets. In this way, the commissure window 342, and thus the leaflets, are offset axially toward an inflow end (which can also be referred to as an upstream end) of the first row of cells 324. As a result, during operation of the valve, the space for blood flow and/or access via a re-access device through the upper (or outflow) portions 358 of the first row of cells 324 is substantially increased.

For example, in some instances, a length 362 of the upper axial strut 348 can be selected such that an axial distance 364 between the outflow end 308 of the frame 300 and outflow edges of the leaflets at the commissure (or axial position of the upper edge 354, as denoted by the dashed line in FIG. 7) is within a selected range. In some examples, the selected range of the axial distance 364 is in a range of 2-6 mm, 2-4 mm, or 2-3 mm. In some examples, the selected range of the axial distance 364 is in a range of 20-50%, 25-45%, or 30-40% of the total axial distance (or height) 326 (FIG. 5) of the elongated cells 350. In some examples, the length 362 of the upper axial strut 348 can be in a range of 0.75-2.5 mm or 1-2 mm or approximately 1.5 mm. As a result, the upper portion 358 of the cells of the first row of cells 324 can be sized to provide adequate blood flow and/or access via a re-access device.

In some examples, outflow edges of the leaflets, away from the commissure (and commissure tabs, toward a center of the valve (toward the central longitudinal axis of the frame)) can be higher (closer to the outflow end 308) or lower than the outflow edges of the leaflets at the commissure tabs.

In some examples the outflow edges of the leaflets, away from the commissure (and commissure tabs, toward a center of the valve (toward the central longitudinal axis of the frame)) can also be offset from the outflow end 308 of the frame 300 by the axial distance 364.

FIG. 8A shows another example for the window strut portions forming the commissure windows 242 of the frame 300. As shown in FIG. 8A, in some examples, the commissure window 242 can be formed by axially extending window strut portions 370a and 370b (e.g., of an axially extending strut) which extend between angled struts 310 at the outflow end 308 and angled struts 234. In some examples, the window strut portions 370a and 370b are formed as one piece and can be collectively referred to as an axially extending window strut or window strut portion that forms the commissure window 242. The window strut portions 370a and 370b can be similar to the window strut portions 240 shown in FIG. 5, but they are configured such that the commissure window 242 which they define is moved lower, toward the angled struts 234 (and the inflow end of the valve). For example, as shown in FIG. 8A, the window strut portions 370a and 370b can form a first or upper end portion 372 above the commissure window 242 (e.g., the end portion that is closer to the outflow end 308) and a second or lower end portion 374 below the commissure window 242 (e.g., the end portion that is further away from the outflow end 308), where the upper end portion 372 is larger (or thicker) in the axial direction than the lower end portion 374. As a result, in some examples, the upper end portion 372 can include an aperture 376. In some examples, the aperture 376 can be configured to receive one or more fasteners (e.g., sutures) when securing commissure tabs of the leaflets to the frame, within the commissure window 242.

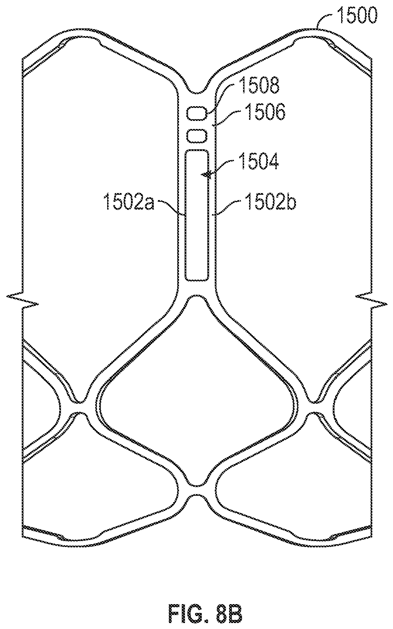

In some examples, the upper end portion 372 can include more than a single aperture 376, such as two or more apertures. For example, FIG. 8B shows another exemplary configuration of window strut portions 1502a, 1502b (which make up an axially extending strut) forming a commissure window 1504 of a frame 1500 of a prosthetic heart valve. An upper end portion 1506 (which can also be referred to herein as an "outflow end portion") of the window strut portions 1502a, 1502b (disposed above the commissure window 1504 in FIG. 8B) includes two apertures 1508 disposed therein. The frame 1500 can be similar to one of the frames disclosed herein, such as the frame 400 or 500 (as described further below). However, the configuration of the window strut portions 1502a, 1502b and the upper end portion 1506 including the two apertures 1508 can be included in many different frames, such as any of the frames disclosed herein. The two apertures 1508 can be configured to receive one or more sutures or alternate fasteners used to secure commissure tabs of adjacent leaflets within the commissure window 1504. In some instances, by using two apertures 1508 (instead of just one), the commissure tabs (forming a commissure) can be more easily and reliably secured to the commissure window 1504.

Returning to FIGS. 5 and 6, in some examples, forming a thinned region across the apex 304, can result in high stresses developed at the center of the thinned region (e.g., the mid-point of the apex), which can decrease a strength of the apex. For example, for the frame 300 with the apices 304 (FIGS. 5 and 6), the point of maximal stress during radial expansion of the frame 300 can be experienced at the apices 304, as denoted by the dashed circle 330 in FIG. 6. In some examples, the maximal stress at the point 330 can be up to 2,140 MPa. It may be desirable to provide a frame for a prosthetic heart valve where the maximal stress experienced during expansion does not exceed 2,000 MPa. In some examples, it is desirable for the maximal stress experienced by the frame during expansion to be less than 1,700 MPa.

Thus, there is a need to provide a reduced height apex or apex region, similar to the apices 304 shown in FIGS. 5 and 6, but that reduces strain concentrations at the apex region and/or moves maximal stresses experienced during radial expansion of the frame away from the apices.

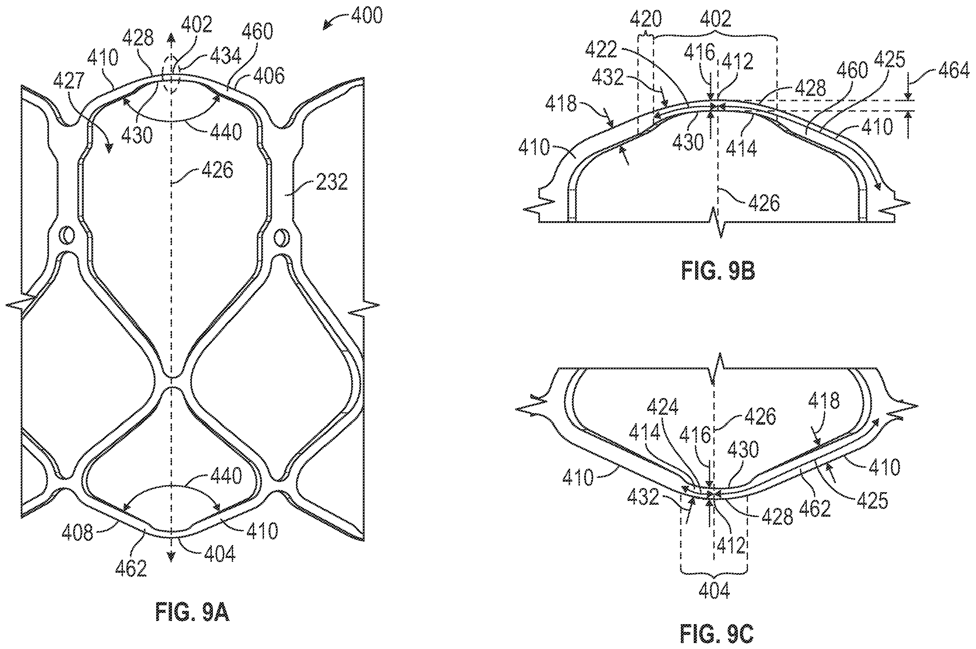

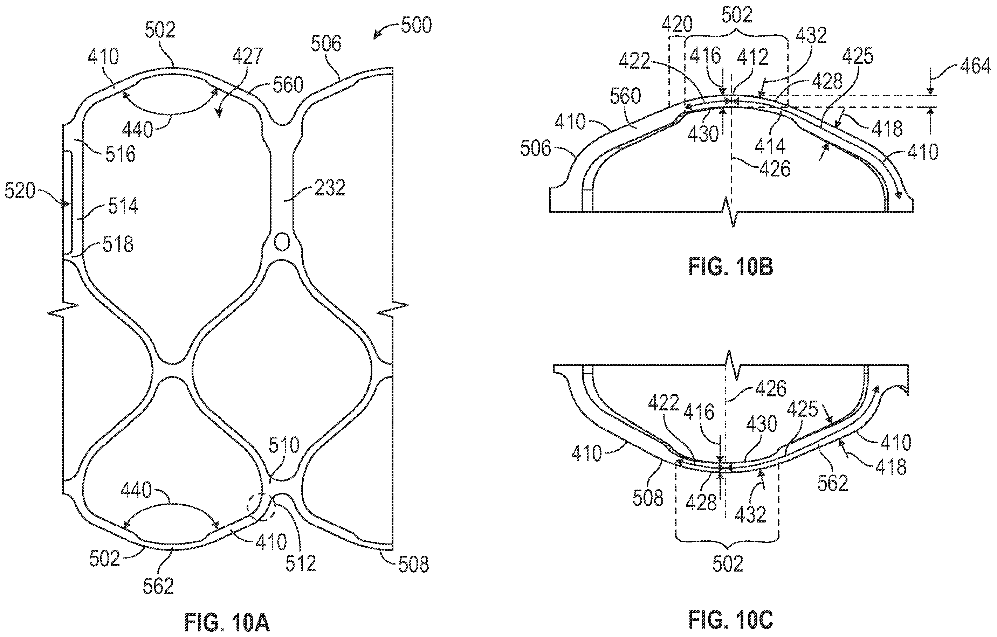

In one example, the strain concentrations and/or maximal stresses experienced at the apices of frames with reduced height apices or apex regions having thinned regions (such as those shown in FIGS. 5 and 6) can be reduced by altering the apex regions of the frame such that they have a reduced width (relative to angled struts of the frame) which extends for a greater length of the inflow or outflow strut comprising the apex region (e.g., at least 25% of a length of the inflow or outflow strut). Exemplary examples of such frame configurations are shown in FIGS. 9A-9C and 10A-10C, as described below.

FIGS. 9A-9C show an example of a portion of frame 400 with outflow apex regions 402 (one shown in FIGS. 9A and 9B) at an outflow end 406 of the frame 400 and inflow apex regions 404 (one shown in FIGS. 9A and 9C) at an inflow end 408 of the frame 400. In some examples, the outflow end 406 can comprise a plurality of outflow struts 460, each outflow strut 460 comprising one outflow apex region 402 and two angled strut portions 410 (which can be referred to as angled struts and can be similar to the angled struts 310 of frame 300, as described above with reference to FIGS. 5 and 6) at the outflow end 406 of the frame 400. For example, each outflow apex region 402 can curve between two angled strut portions 410, with each strut portion extending between the outflow apex region 402 and a different axial strut 232 (as shown in FIG. 9A) or window strut portion. Similarly, the inflow end 408 can comprise a plurality of inflow struts 462, each inflow strut 462 comprising one inflow apex region 404 and two angled strut portions 410 at the inflow end 408 of the frame 400.

Each of the outflow apex regions 402 and inflow apex regions 404 can comprise an apex 412 (the highest or most outward extending, in an axial direction, point) and thinned (or narrowed) struts portions 414 extending from either side of the apex 412 and connecting to corresponding angled strut portions 410 (FIGS. 9B and 9C). In this way, each of the outflow apex regions 402 and inflow apex regions 404 can form a narrowed transition region between and relative to the two angled strut portions 410 extending from the corresponding apex region.

In some examples, each of the outflow apex regions 402 and inflow apex regions 404 can include transition portions 420 (FIG. 9B) that narrow or taper in width from the corresponding angled strut portion 410 to the corresponding thinned strut portion 414.

In other examples, the outflow apex regions 402 and inflow apex regions 404 may not comprise the transition portions 420, and instead, the transition portions 420 can be included in the corresponding angled strut portions 410.

The thinned strut portions 414 of the outflow apex region 402 and the inflow apex region 404 can have a width 416 that is smaller than a width 418 of the angled strut portions 410 (FIGS. 9B and 9C). In some examples, the width 416 can be a uniform width (e.g., along an entire length of the strut portion 414). As introduced above, the width of a strut portion (as used herein with reference to width 416 of the thinned strut portions 414 and the width 418 of the angled strut portions 410) is measured between opposing locations on opposing surfaces of a strut portion that extend between the radially facing inner and outer surfaces of the strut portion.

In some examples, the width 416 of the thinned strut portions 414 can be from about 0.06 - 0.15 mm smaller than the width 418 of the angled strut portions 410. For example, in some instances, the width 418 of the angled strut portions 410 can be about 0.3 mm and the width 416 of the thinned strut portions 414 can be in a range of about 0.15 - 0.24 mm. In some examples, the width 416 of the thinned strut portions 414 can be in a range of about 0.18 - 0.22 mm. In some examples, the width 416 of the thinned strut portions 414 can be about 0.2 mm (e.g., ± 0.03 mm). The width 416 can also be referred to as a width of the outflow apex region 402 and the inflow apex region 404.

In contrast to the apices of the frame 300 (FIGS. 5 and 5), the outflow apex regions 402 and inflow apex regions 404 of the frame 400 (FIGS. 9A-9C) have the thinned strut portions 414 which extend for a greater distance between the angled strut portions 410. For example, the thinned strut portions 414 of the outflow apex regions 402 can have a first length 422 (FIG. 9B). In some examples, the first length is in a range of 0.8-1.4 mm, 0.9-1.2 mm, or 0.95-1.05 mm. In some examples, the first length is about 1.0 mm (e.g., ±0.03 mm). Thus, thinned strut portions 414 on and extending from either side of the apex 412 of the outflow apex region 402 can have the first length 422 (only one first length 422 shown in FIG. 9B). Said another way, each outflow apex region 402 can include two thinned strut portions 414 having the first length 422, each extending from the apex 412, outward relative to a central longitudinal axis 426 of the cells. Thus, a total length of the apex region 402 can be two times the first length 422 (e.g., in a range of 1.6-2.8 mm, 1.8-2.4 mm, or 1.9-2.2 mm, or about 2.0 mm).

Further, in some examples, the thinned strut portions 414 of the inflow apex regions 404 can have a second length 424, where the second length 424 is smaller than the first length 422 (FIG. 9C). In some examples, the second lengths 424 is in a range of 0.3-0.7 mm, 0.4-0.6 mm, or 0.45-0.55 mm. In some examples, the second length 424 is about 0.5 mm (e.g., ±0.03 mm). Each of two thinned strut portions 414 of the same inflow apex region 404 can have the second length 424 (only one indicated in FIG. 9C). Thus, a total length of the apex region 404 can be two times the second length 424 (e.g., in a range of 0.6-1.4 mm, 0.8-1.2 mm, or 0.9-1.1 mm, or about 1.0 mm).

Each outflow strut 460 and inflow strut 462 can have a length that includes an apex region (402 or 404), the transition portions or regions 420, and the two angled strut portions 410 on either side of the apex region. One half the total length of each outflow strut 460 and inflow strut 462 is shown in FIGS. 9B and 9C as length 425, which extends from an end of one angled strut portion 410 to the central longitudinal axis 426. Thus, the length of each outflow strut 460 and inflow strut 462 is two times length 425. In some examples, the length 425 for half of each inflow strut 462 can be different than the length 425 for half of each outflow strut 460.

The length of each thinned strut portion 414 can be at least 25% of the length 425 of the corresponding half outflow strut 460 or inflow strut 462. Said another way, the length of each outflow apex region 402 (a total length being two times the first length 422) and each inflow apex region 404 (a total length being two times the second length 424) can be at least 25% of the total length (two times length 425) of the outflow strut 460 or inflow strut 462. In some examples, the length of each apex region (such as the outflow apex region 402) can be more than 25% of the total length of the corresponding outflow strut 460 or inflow strut (two times length 425), such as 25-35%.

In some examples, each outflow apex region 402 and inflow apex region 404 can comprise a curved, axially facing outer surface 428 and an arcuate or curved, axially facing inner depression 430 which forms the thinned strut portions 414. For example, the curved inner depression 430 can depress toward the curved outer surface 428 from an inner surface of the angled strut portions 410, thereby forming the smaller width thinned strut portions 414. Thus, the curved inner depressions 430 can be formed on a cell side of the apex region (e.g., as opposed to the outside of the apex region).

In some examples, the curved outer surface 428 of each apex region (402 or 404) can form a single, continuous curve from one angled strut portion 410 on a first side of the apex region to another angled strut portion 410 on an opposite, second side of the apex region. In contrast, the apices of the frames of the prosthetic heart valves 10 and 200 shown in FIGS. 1 and 3, respectively, can include three or four curves (e.g., apex 220 has four bends or curves from one angled strut on one side of the apex to another angled strut on an opposing side of the apex).

Each outflow apex region 402 and inflow apex region 404 can have a radius of curvature 432, along the curved outer surface 428 (e.g., in some instances, along an entirety or an entire length of the curved outer surface 428). The radius of curvature 432 at the apex 412 and/or along the entire curved outer surface 428 of the apex region can be larger than previous apex designs with more pointed or U-shapes. In some examples, the radius of curvature 432 can be greater than 1 mm. In some examples, the radius of curvature 432 can be in a range of 1-20 mm, 3-16 mm, or 8-14 mm. In some examples, the radius of curvature 432 can be greater than 10 mm. In some examples, the radius of curvature 432 can be about 13.5 mm (±0.03 mm). The radius of curvature 432 can be dependent on (and thus change due to changes in) the width 416 (e.g., the amount of reduction in width from the angled strut portions 410) and the length (422 or 424) of the thinned strut portions 414.

In some examples, the radius of curvature 432 can be defined such that the apex region (inflow apex region 404 or outflow apex region 402) is flat (e.g., the radius of curvature 432 can be infinite). For example, in such instances, the outer surface 428 can be planar, with the planar outer surface 428 defined normal to the central longitudinal axis 426.

Further, a height (an axial height) 464 of the outflow apex regions 402 and inflow apex regions 404, which can be defined in the axial direction from an outer surface of the two angled strut portions 410 to the curved outer surface 428 of the apex region at the apex 412, can be the width 416 of the thinned strut portions 414 (as shown in FIG. 9B). In this way, the height 464 of the outflow apex regions 402 and the inflow apex regions 404 can be relatively small and not add much to the overall axial height of the radially expanded frame 400. Thus, as described above, leaflets secured to the frame 400 can be disposed closed to the inflow end 408, thereby leaving a larger open space at the outflow end 406 of the frame 400 that is not blocked by the leaflets.

A remainder of the frame 400 can be similar to the frame 300 of FIGS. 5 and 6. In some examples, each of the outflow apex regions 402 and the inflow apex regions 404 can form an angle 440 between the two angled strut portions 410 extending from either side of the corresponding apex region (FIG. 9A). In some examples, the angle 440 can be similar to the angle 320 of frame 300. For example, in some instances, the angle 440 can be in a range of 120 (not inclusive) to 140 degrees (e.g., such that the angle 440 is greater than 120 degrees and less than or equal to 140 degrees). In some examples, the angle 440 can be in a range of 135-140 degrees, 138-140 degrees, or 139-140 degrees. In some examples, the angle 440 can be about 140 degrees (e.g., ±1 degree).

The point of maximal stress (during expansion of the frame 400) of the frame 400, with the outflow apex regions 402 and inflow apex regions 404 described above, can be experienced at the outflow apex regions 402, as denoted by the dashed circle 434 in FIG. 9A. In some examples, the maximal stress at the point 434 can be 1,795 MPa. While this is smaller than the maximal stress of the frame 300 (FIG. 6), it may still be desirable to have a frame with a lower maximal stress, which is located away from the apex regions of the frame.

FIGS. 10A-10C show another example of a portion of a frame 500 with apex regions 502 at both an inflow end 508 and outflow end 506 of the frame 500. The apex regions 502 can be the same or similar to the outflow apex regions 402 of frame 400. For example, each apex region 502 (at the inflow end and outflow end) can comprise the apex 412 and two thinned strut portions 414, one thinned strut portion 414 extending from either side of the apex 412 to a corresponding, wider, angled strut portion 410. Further, similar to the apex regions 402 of frame 400, each apex region 502 at the inflow end 508 and outflow end 506 of the frame 500 can have the width 416 and thinned strut portions with first length 422.

Further, each apex region 502 and two corresponding angled strut portions 410 at the outflow end 506 can form an outflow strut 560 and each apex region 502 and two corresponding angled strut portions 410 at the inflow end 508 can form an inflow strut 562.

Similar to frame 400, the first length 422 of the thinned strut portion 414 can result in a total length of the apex region 502 being at least 25% of a total length (two times length 425 shown in FIGS. 10B and 10C) of the corresponding outflow strut 560 or inflow strut 562. In some examples, the length of each apex region 502 can be more than 25% of the total length (two times length 425) of the outflow strut 560 or inflow strut 562, such as 25-35% (FIGS. 10B and 10C).

Thus, for frame 500, both the apex regions 502 at the inflow end 508 and outflow end 506 can have the longer thinned strut portions 414 with the first length 422. This configuration of the apex regions 502 can result in a reduced maximal stress, at a location spaced away from the apex regions 502. For example, the point of maximal stress (during expansion of the frame 500) of the frame 500 can be experienced at a region of the angled strut portions 410, proximate to a strut junction 510 and away from the apex region 502, as denoted by the dashed circle 512 in FIG. 10A. In some examples, the maximal stress at the point 512 can be 1,619 MPa.

In some examples, the apex regions 502 of the frame 500 can be further defined by the angle 440 and the radius of curvature 432 (FIG. 10A), as described above with reference to FIGS. 9A-9C.

In some examples, the frame 500 can comprise axially extending window strut portions 514 defining commissure windows 520 of the frame 500 (FIG. 10A). The window strut portions 514 can form an upper (or outflow) end portion 516 above the commissure window 520 and a lower (or inflow) end portion 518 below the commissure window 520, where the upper end portion 516 is larger (or longer, in the axial direction) than the lower end portion 518. This configuration can offset the commissure window 520 slightly toward the inflow end 508 of the frame 500 (similar to as described above with reference to the window strut portions 370a and 370b in FIG. 8).

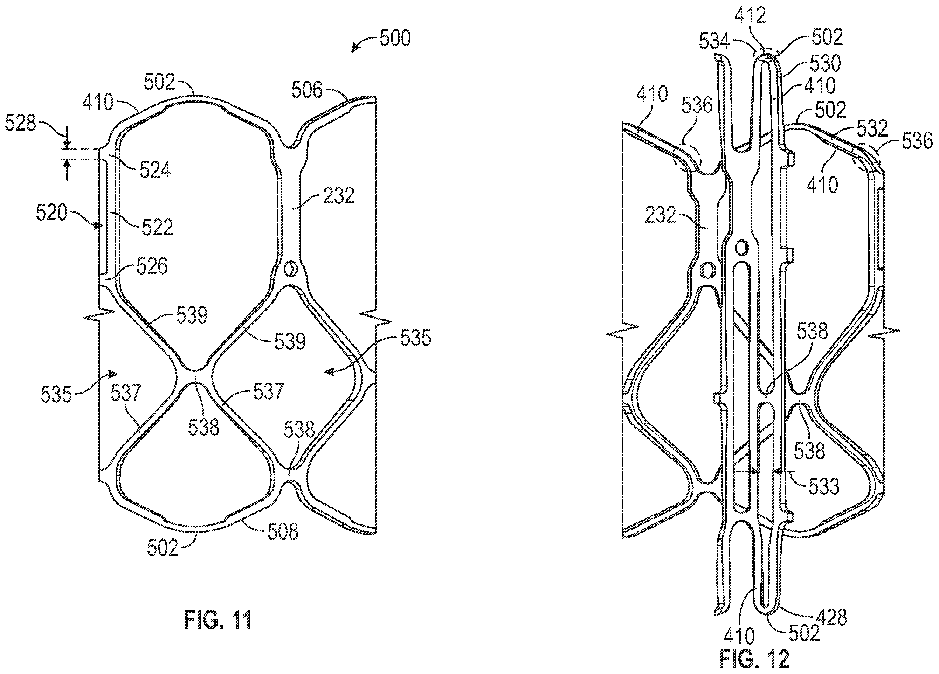

In other examples, as shown in the partial frame view of FIG. 11, the frame 500 can comprise axially extending window strut portions 522 defining the commissure windows 520 of the frame 500. The window strut portions 522 can form an upper (outflow) end portion 524 above the commissure window 520 and a lower (inflow) end portion 526 below the commissure window 520, where a length 528 (in the axial direction) of the upper end portion 524 is the same or substantially the same as the lower end portion 526 (FIG. 11). In some examples, the length 528 of the upper end portion 524 and lower end portion 526 can be larger than the width 418 of the struts of the frame (e.g., the angled strut portions 410). For example, in some instances, the length 528 can be in a range of 0.35-0.5 mm or 0.38-0.45 mm, or approximately 0.4 mm (e.g., ±0.03 mm).

The reduction in maximal stress during radial expansion of the frame 500 (compared to previously disclosed frames, including frames 300 and 400), away from the apex regions 502, can be attributed to stress stiffening of the apex regions 502 which occurs during radially compressing or crimping the frame 500 into its radially compressed configuration. For example, as shown in FIG. 12, the frame 500 can be radially compressed or crimped into a radially compressed configuration 530 (e.g., for delivery on the distal end portion of a delivery apparatus to a target implantation site) and then (e.g., upon reaching the target implantation site for implantation) radially expanded to its radially expanded configuration 532. FIG. 12 shows overlapping, partial views of the frame 500 in its radially compressed configuration 530 and its radially expanded configuration 532 for comparison purposes.

As an example, when the frame 500 is crimped into its radially compressed configuration 530, the apex regions 502, particularly around a more central region including the apex 412, bend and are plastically deformed. As a result, the apex regions 502 are strain hardened (or stress stiffened). As shown in FIG. 12, the points of maximal stress during crimping occur at the apex regions 502, as denoted by dashed circle 534.

Then, upon subsequent radial expansion to the radially expanded configuration 532, since the apex regions 502 have been strain hardened, they will not plastically deform during expansion. Instead, the points of maximal stress during expansion of the frame 500 can occur along the angled strut portions 410, away from the apex regions 502, as denoted by dashed circles 536 in FIG. 12. In this way, the bending points of the frame between crimping and expansion of the frame 500 are shifted from the apex regions 502 to points away from the apex regions 502 (points shown by circles 536), respectively. As a result, the apex regions 502 can be stronger and less likely to experience degradation and the frame 500 can be more robust.

As shown in FIGS. 11 and 12, the frame 500 can also comprise horizontal struts 538 that extend between adjacent cells 535 of a row of cells of the frame 500. The horizontal struts 538 can extend in a circumferential direction and also be referred to as circumferentially extending struts 538. The horizontal struts 538 can connect angled struts of two adjacent rows of angled struts of the frame 500 to one another. For example, each horizontal strut 538 can connect to two angled struts of one row of struts (e.g., struts 537 shown in FIG. 11) and two angled struts in another, adjacent row of struts (e.g., struts 539 shown in FIG. 11). As a result, an angled strut 539 extending between a commissure window 520 and the horizontal strut 538 and an angled strut 537 extending between the horizontal strut 538 and another horizontal strut 538 disposed adjacent to the inflow end 508 of the frame can be aligned along an angled line that can follow a scallop line of the leaflets (when the leaflets are attached to the frame 500). Thus, the horizontal struts 538 can allow the angled struts to follow a shape that more closely matches a shape of the scallop line of the leaflets when the frame 500 is in the radially expanded configuration 532. Additionally, the horizontal struts 538 can serve as spacers that can maintain a gap 533 between the angled struts when the frame 500 is in the radially compressed configuration 530 (as shown in FIG. 12), thereby reducing a risk of pinching the leaflets between the struts in the radially compressed configuration 530.

FIG. 13 is a perspective view of an example of a prosthetic heart valve 600 comprising a frame 620 and an outer (fabric) skirt 602 secured to the frame 620. The frame 620 can comprise a plurality of interconnected and angled struts 630 and a plurality of apex regions 632 at an inflow end 622 and outflow end 624 of the frame 620. The frame 620 can be similar to (or replaced by) any one of the frames described herein, such as one of frame 300 (FIGS. 5-8), frame 400 (FIGS. 9A-9C), or frame 500 (FIGS. 10A-10C). As shown in FIG. 13, the outer skirt 602 can extend around an outer surface of the frame 620, from the inflow end 622 toward the outflow end 624 (covered by the outer skirt 602 in FIG. 13). In some examples, as shown in FIG. 13, the outer skirt 602 can be secured to struts of the frame at the inflow end 622 by one or more sutures 604.

Further, in some examples, an outflow end of the outer skirt 602 can be secured to inflow end portions 248 of the axial struts 232 and/or to upper or outflow ends of the angled struts 234 (see FIG. 5) by one or more sutures 606. In some examples, a portion of the sutures 606 can extend through and be secured to the apertures 249 in the inflow end portions 248 of the axial struts 232 (apertures 249 shown in dashed lines in FIG. 13 to denote their position underneath the outer skirt 602). In some examples, the sutures 606 and/or the sutures 604 can be in-and-out stitches.

In some examples, as shown in FIG. 13, additional sutures 608, which can be configured as whip stitches, can secure the outer skirt 602 to angled struts of the frame 620 disposed and extending between the inflow end 622 and inflow ends of window strut portions forming the commissure windows of the frame 620.

As introduced above, exposed apices of a frame of a prosthetic heart valve can interact with an inflatable balloon of a delivery apparatus on which the prosthetic heart valve is mounted (radially compressed around) and/or a delivery sheath through which the delivery apparatus is advanced en route to a target implantation site. As such, exposed apices of the prosthetic heart valve frame have the potential to be traumatic to the delivery apparatus, delivery sheath, and/or a patient's vasculature. The apex regions (402, 404) of the frame 400 (FIGS. 9A-9C) and the apex regions 502 of the frame 500 can be more atraumatic than previously disclosed apices having sharper points or U-shapes due to their more round-shaped crimped profile (which may be provided by the increased angle and shape of the apex regions, as described above).

For example, as shown in FIG. 12, when the frame 500 is in the radially compressed configuration 530, the apex regions 502 form a curved shape along their outer surface 428. Such a shape my facilitate easier sliding over the inflatable balloon of the delivery apparatus when the prosthetic heart valve is pushed from an initial mounting location (e.g., off or partially off the balloon) to a deployment location over the balloon (e.g., when the prosthetic heart valve is originally crimped off a majority portion of the balloon). The continuously curved shape of the apex regions of the frame may also reduce the likelihood of the apex regions (e.g., at the apex 412) penetrating into the balloon (when initially mounted on the balloon, as shown in the exemplary delivery apparatus of FIG. 2) and/or into the delivery sheath during delivery to the target implantation site.

In some examples, it may be desirable to provide an even more atraumatic surface at the apex regions (e.g., at least at the distal end of the frame, which can be the inflow end). In one example, the reduced height, curved, and thinner apex regions (e.g., apex regions 402, 404, and/or 502) can be further rounded and/or filleted at their edges (e.g., the outer edge forming the curved outer surface 428 shown in FIGS. 9B, 9C, 10B, 10C, and 12).

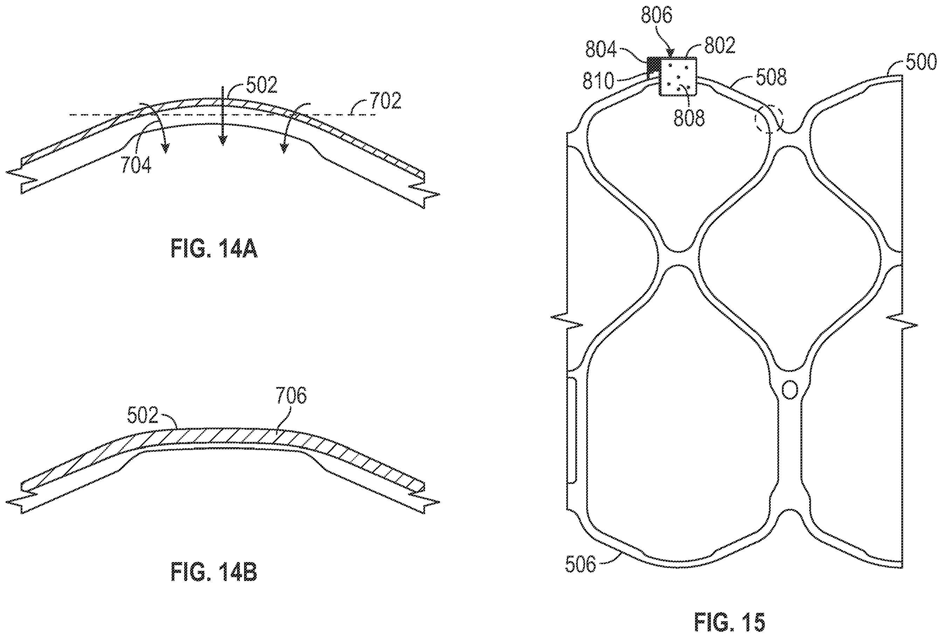

In another example, as shown in FIGS. 14A and 14B, the exemplary apex region 502 (or alternatively apex regions 402 or 404) shown in FIG. 14A can be rotated or twisted about its axis 702 (which may be referred to as a transverse axis), in the direction of arrows 704, to form a twisted outer surface 706 (FIG. 14B). Such a twisted outer surface 706 may be less blunt and more atraumatic to the delivery apparatus components and/or native anatomy that is exposed to or in contact with the apex region 502.

In another example, at least a portion of one or more apex regions (e.g., apex regions 402, 404, and/or 502) of a frame (e.g., one of frames 400 or 500) can be covered by cushioning elements. For example, in some instances, as shown in FIG. 15, a cushioning element 802 can cover at least a portion of an apex region 502 at the inflow end 508 of the frame 500. In other examples, the frame 500 can be replaced with frame 400 (FIGS. 9A-9C) and the cushioning elements 802 can cover the inflow apex regions 404 and/or the outflow apex regions 402.

As shown in FIG. 15, the cushioning element 802 can be coupled to and cover at least a portion (e.g., a distal end, such as the apex 412) of an apex region 502 at the inflow end 508 of the frame 500. In some examples, each apex region 502 at the inflow end 58 can be covered, at least in part, by a different cushioning element 802.

The cushioning element 802 can comprise a flexible material folded over the apex region 502 (e.g., a central portion of the apex region 502 including the apex 412). In some examples, the flexible material may be cloth. In other examples, the flexible material may be another type of relatively soft, flexible material such as fabric, a relatively soft, flexible polymer (e.g., silicone), or the like. When made from a fabric, the fibers of the fabric can be made of any of various biocompatible materials, such as polyethylene terephthalate (PET). The fabric can be a woven fabric, a non-woven fabric, or a pile fabric (e.g., velvet, velour, etc.) having tufts or loops of fibers extending from a woven base layer. Any of the various fabrics disclosed in U.S. Publication No. 2019/0192296 can be used to form the cushioning element 802.

In some examples, the cushioning element 802 comprises a plurality of folds 804 arranged over and extending across the curved outer surface 428 of the apex region 502 and forming a distal surface (or layer) 806 of the cushioning element 802. In this way, the plurality of folds 804 can be formed over and across the apex 412 of the apex region 502. The plurality of folds 804 can extend between an inner layer 808 and an outer layer 810 of the cushioning element 802, where the inner layer 808 covers a radially inward facing inner surface (e.g., toward a central longitudinal axis of the frame) of the corresponding apex region 502 and the outer layer 810 covers a radially outward facing surface (e.g., away from a central longitudinal axis of the frame) of the corresponding apex region 502. In this way, the inner layer 808 is arranged closer to the central longitudinal axis than the outer layer 810.

In some examples, the inner layer 808 and outer layer 810 of the cushioning element 802 can extend along/across the entire width 416 of the apex region 502.

By covering at least the curved outer surface 428 of the apex region 502, at the apex 412, the cushioning element 802 may further reduce abrasion between the exposed apex regions 502 and an inflatable balloon of a delivery apparatus and/or an inner surface of a sheath during delivery of the prosthetic heart valve to the target implantation site, thereby reducing degradation to the sheath and/or the balloon.

It should be understood that references to the distal and proximal ends of the prosthetic valve and/or frame 500 can refer to the positions of the ends of the valve during delivery on the delivery apparatus. For example, when a prosthetic heart valve comprising the frame 500 (or frame 400) is mounted on the distal end portion of the delivery apparatus (such as depicted in FIGS. 2 and 4), the inflow end of the prosthetic valve is the distal-most end of the prosthetic valve and the outflow end of the prosthetic valve is the proximal-most end of the prosthetic valve. This arrangement can be suitable for retrograde delivery of the prosthetic heart valve through the aorta to the native aortic valve. However, in other examples, the outflow end of the prosthetic heart valve can be the distal end of the prosthetic heart valve during delivery, depending on the particular delivery approach and the particular implantation location within the heart. For example, when delivering a prosthetic heart valve to the native mitral valve via a trans-septal delivery path, the outflow end of the prosthetic heart valve can be the distal-most end of the prosthetic valve. Thus, the cushioning elements 802 (and/or differently configured cushioning elements) can be mounted on the inflow end or the outflow end of the prosthetic valve, depending on the particular delivery approach and the particular implantation location within the heart for the procedure.

Further, in other examples, the cushioning elements 802 can be mounted on both the inflow end and the outflow end of the prosthetic heart valve. In still other examples, the cushioning elements 802 can be mounted only on the apex regions 502 at the inflow end of the prosthetic heart valve.

In some examples, as shown in FIG. 15, each apex region 502 (e.g., at least at the inflow end or distal end of the frame during delivery) of the plurality of apex regions 502 includes a discrete cushioning element 802 covering at least the apex 412 of the apex region 502. In alternate examples, a single cushioning element may cover each and every apex region 502 of the plurality of apex regions 502 at the inflow end 508 of the frame 500. For example, in these instances, the single cushioning element may comprise a single, circumferential sleeve, arranged around an entirety of the circumference of the inflow end 508 and covering all of the apex region 502 at the inflow end 508. In other examples, the frame 500 may include two or more cushioning elements, each covering at least two apex regions 502. In still other examples, the cushioning element or elements may be part of an external skirt or an internal skirt of the valve. In these examples, the skirt may have integral individual cushioning elements or a single cushioning element.

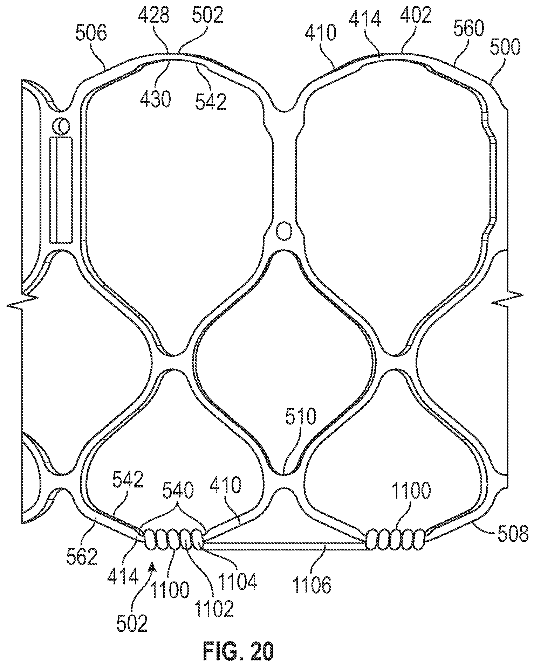

FIGS. 20-29 present additional examples for a covering and/or a cushioning element for apex regions of a frame of a prosthetic heart valve. These coverings and/or cushioning elements can, for example, reduce friction between the apex regions and one or more components of a delivery apparatus or system, such as an introducer or introducer sheath, a delivery sheath, and/or a balloon of the delivery apparatus, e.g., when the prosthetic heart valve is radially compressed around a portion of a distal end portion of the delivery apparatus and/or being advanced relative to the delivery sheath (e.g., to expose the prosthetic heart valve at an implantation site). Further, in some examples, the delivery sheath through which the radially compressed prosthetic heart valve on the delivery apparatus is advanced through can be an expandable delivery sheath. As such, during advancement through the expandable delivery sheath, the prosthetic heart valve can apply a radial force to expand the sheath (e.g., against the vascular wall), as well as an axial force component resulting from the contact of the frame (e.g., the inflow apices or apex regions of the frame) against the inner wall of the sheath during advancement (e.g., shear stress). This axial force component is proportional to the friction coefficient between the apices (or apex regions) and the delivery sheath, and the stress is inversely proportional to the area (force divided by contact area). Thus, reducing the coefficient of friction and increasing contact area at the distal end (e.g., inflow end when using a transfemoral delivery approach with a prosthetic aortic valve) of the prosthetic heart valve may be desirable to reduce the axial force component during valve advancement through the delivery sheath.

FIGS. 20-24 show examples of wrapping one or more apex regions of a frame of a prosthetic heart valve with a material. These wrappings can, for example, reduce friction, provide cushioning, and/or a non-abrasive covering to the apex regions. The wrappings can be formed of various materials such as a polymeric or fabric material. For example, one or more apex regions (e.g., apex regions 402, 404, and/or 502) of a frame (e.g., one of frames 400 or 500) can be covered by a covering element that is looped or wrapped around a circumference of the strut portions forming the apex region. In some examples, as shown in FIG. 20, a covering element 1100 (e.g., a suture or another polymeric or fabric strip or band of material) can cover and/or be wrapped around at least a portion of an apex region 502 at the inflow end 508 of the frame 500.

It should be noted that the frame 500 is used in FIGS. 20-24 by way of example and, in other examples, the covering element 1100 can cover an apex region or apex of any of the other frames described herein in a manner similar to that described below with reference to FIGS. 20-24.

As introduced above, each apex region 502 can comprise an apex 412 and two thinned strut portions 414, one thinned strut portion 414 extending from each side of the apex 412 to a corresponding, wider, angled strut portion 410 of the inflow strut 562 (or outflow strut 560). Further, each apex region 502 can comprise a curved, axially facing outer surface 428 and an inner depression 430 which forms the thinned strut portions 414. In some examples, the curved outer surface 428 of each apex region 502 can form a single, continuous curve from one angled strut portion 410 on a first side of the apex region 502 to another angled strut portion 410 on an opposite, second side of the apex region 502.

Thus, each apex region 502 can comprise shoulders 540 (or shoulder portions) on both sides or ends of the apex region 502 that transition from the narrower thinned strut portions 414 of the apex region 502 to the wider angled strut portions 410 of the corresponding inflow strut 562 or outflow strut 560. The shoulders 540 can be disposed on/part of an axially facing inner surface 542 of the inflow strut 562 or outflow strut 560.

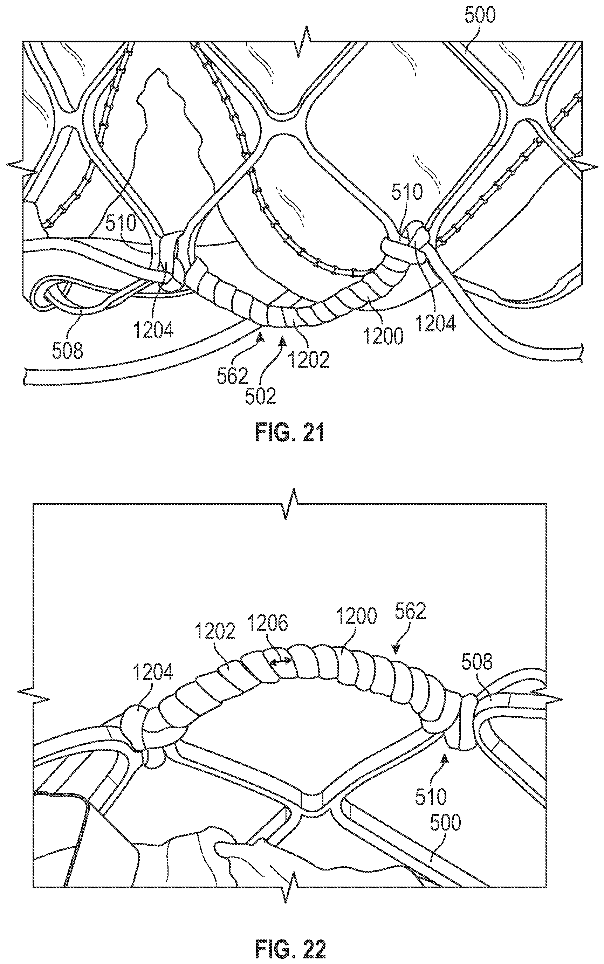

As shown in FIG. 20, the covering element 1100 can wrap around and cover at least a portion of the apex region 502. In some examples, the covering element 1100 can wrap around and cover a partial section or the entire thinned strut portions 414 between the shoulders 540 of the apex region 502. In other examples, the covering element 1100 can wrap around and cover the apex region 502 and a portion of one or more of the angled strut portions 410 connected to the apex region 502 (e.g., as shown in FIGS. 21-24 and described further below).

In some examples, a covering element 1100 can wrap around and cover the apex regions 502 of one or more of the inflow struts 562 (e.g., in some instances, each inflow strut 562). In some examples, a covering element 1100 can wrap around and cover the apex regions 50 of one or more of the outflow struts 560.

The covering element 1100 can be configured as a strip or band of material that is looped (or wrapped) around the apex regions 502. The covering element 1100 can be wrapped around a circumference of the thinned strut portions 414 of the apex region 502 such that a plurality of loops 1102 are formed adjacent to one another across the apex region 502 (e.g., relatively tight loops such that the loops 1102 are contacting and not hanging off the thinned strut portions 414). In some instances, adjacent loops can at least slightly overlap each other. In other instances, adjacent loops can abut one another (i.e., non-overlapping). In yet other instances, adjacent loops can comprise a gap therebetween where the apex region is slightly exposed.

The covering element 1100 can comprise a relatively smooth material such as polytetrafluoroethylene (PTFE). In some examples, the covering element 1100 can be a strip or monofilament comprising a higher strength material that has a relatively low friction coefficient, such as ultra-high-molecular-weight polyethylene (UHMWPE) or polyetheretherketone (PEEK). Such materials can provide a larger radius of curvature which can result in reduced abrasion against the delivery sheath when the radially compressed prosthetic heart valve is being navigated through the delivery sheath to the implantation site by the delivery apparatus.

As shown in FIG. 20, in some examples, the covering element 1100 can be coupled at one end to one shoulder 540 of an apex region 502 (e.g., by knotting or tying the end of the covering element 1100 into a knot such as a "granny" or double knot) and then looped repeatedly around the thinned strut portions 414 of the apex region 502 to the opposite shoulder 540 of the apex region 502. As a result, multiple loops 1102 of the covering element 1100 can be formed across the apex region 502, between the two shoulders 540 of the apex region 502.

In some examples, a remaining or free end portion of the strand of the covering element 1100 can pass below a last (or end) loop 1104 of the covering element 1100 to lock it in position around the apex region 502. From the last loop 1104, the free end portion of the strand of the covering element 1100 can then extend toward the nearest shoulder 540 of the adjacent apex region 502 (FIG. 20), thereby forming a bridge portion 1106. The free end portion of the covering element 1100 can then loop or wrap around the adjacent apex region 502.

In some examples, this process can be repeated to extend a single strip or band making up the covering element 1100 (or one or more strips or bands connected together) around and between all the apex regions 502 at the inflow end 508 (or outflow end 506) of the frame 500. As a result, all the inflow apex regions 502 at the inflow end 508 (and/or outflow end 506) of the frame 500 can be covered by the covering element 1100. In this way, the covering element 1100 can comprise a plurality of loops 1102 around and covering each apex region 502 at the inflow end 508 (and/or outflow end) of the frame 500 and a plurality of bridge portions 1106 (or extension portions) between the loops 1102 of adjacent apex regions 502. The plurality of bridge portions 1106 can extend across the inflow end 508 of the frame, from one plurality of loops 1102 of one apex region 502 to another plurality of loops 1102 of an adjacent apex region. This can result in the covering element 1100 creating a ring of covering material at the inflow end 508, around a circumference of the frame 500.

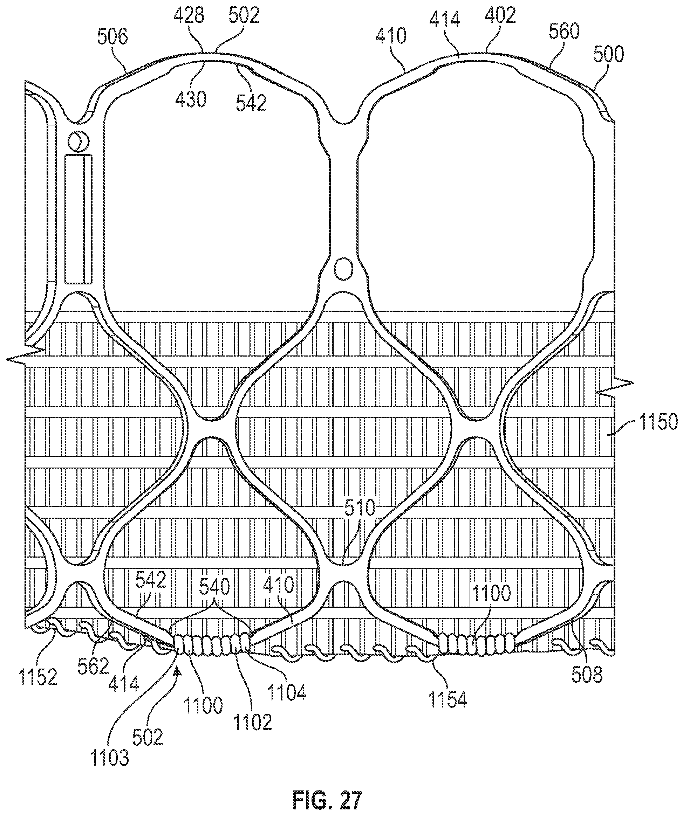

In some examples, as shown in FIG. 27, the covering element 1100 can also extend through an outer skirt disposed around an outer surface of the frame 500, thereby securing the outer skirt to the frame 500. For example, an exemplary outer skirt 1150 that is disposed around an outer surface of the frame 500 can be secured or stitched to an inflow end of the frame with the covering element 1100 (FIG. 27). FIG. 27 shows an interior view of a portion of the frame 500 with the outer skirt 1150 arranged around and secured to the frame (the outer skirt 1150 appears behind the frame 500 in FIG. 27 due to it being an interior view). The outer skirt 1150 shown in FIG. 27 can comprise any combination of the materials described herein with reference to an outer skirt of a prosthetic heart valve. Further, in alternate examples, a different outer skirt can be secured to the frame 500 or another frame of a prosthetic heart valve in a similar manner to as described below with reference to FIGS. 27 and 28A-29 (e.g., the outer skirt 1150 can be replaced by the outer skirt 602 of FIG. 13 or outer skirt of FIG. 1 and/or the frame 500 or 600 can be replaced by the frame 400 of FIG. 9A or frame 300 of FIG. 5).

As shown in FIG. 27, the covering element 1100 can extend around the apex regions 502 of the frame 500 (as described above) and through a material (e.g., fabric) of the outer skirt 1150, along an inflow edge portion 1152 of the outer skirt 1150. In some instances, one or more or each of the loops 1102 of the covering element can wrap around the apex region 502 (as described above) and extend through the outer skirt 1150. From the last loop 1104, the covering element 1100 can then be stitched through the inflow edge portion 1152 of the outer skirt 1150, forming a plurality of whip stitches 1154 through and around the inflow edge portion 1152, between adjacent apex regions 502.

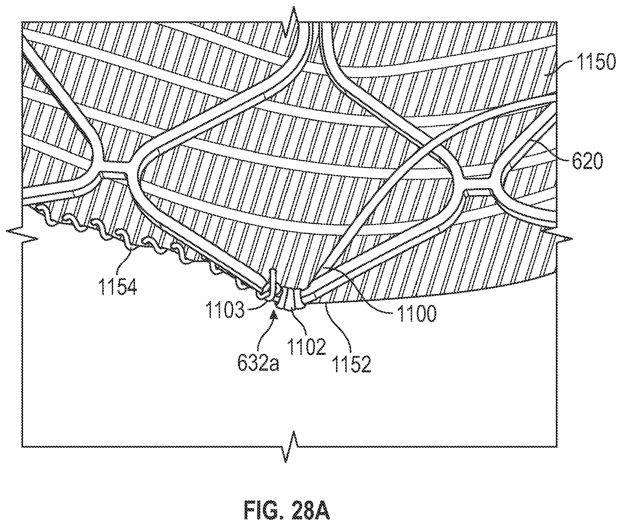

FIGS. 28A-28C show an exemplary method for forming the loops 1102 and whip stitches 1154 and securing the outer skirt 1150 to another exemplary frame 620 with the covering element 1100. Similar to the other frames described herein, the frame 620 can include apex regions 632 (632a and 632b shown in FIGS. 28A-28C) extending between angled struts 630. Turning first to FIG. 28A, starting at a first apex region 632a of the frame 620, the covering element 1100 can be extended through the inflow edge portion 1152 of the outer skirt 1150 (e.g., using a needle) and looped around the first apex region 632a (e.g., adjacent to a first shoulder of the first apex region 632a) to form a first loop 1103. In some instances, the covering element 1100 can pass through the first loop 1103 to form a knot (e.g., a first knotted loop 1103). The covering element 1100 can then be wrapped around the first apex region 632a, adjacent to the first loop 1103, to form a plurality of loops 1102 (which can, in some instances, be whip stitches). In some examples, three loops 1102 can be formed. However, in alternate examples, the number of loops 1102 can be greater or less than three, such as two, four, or the like.

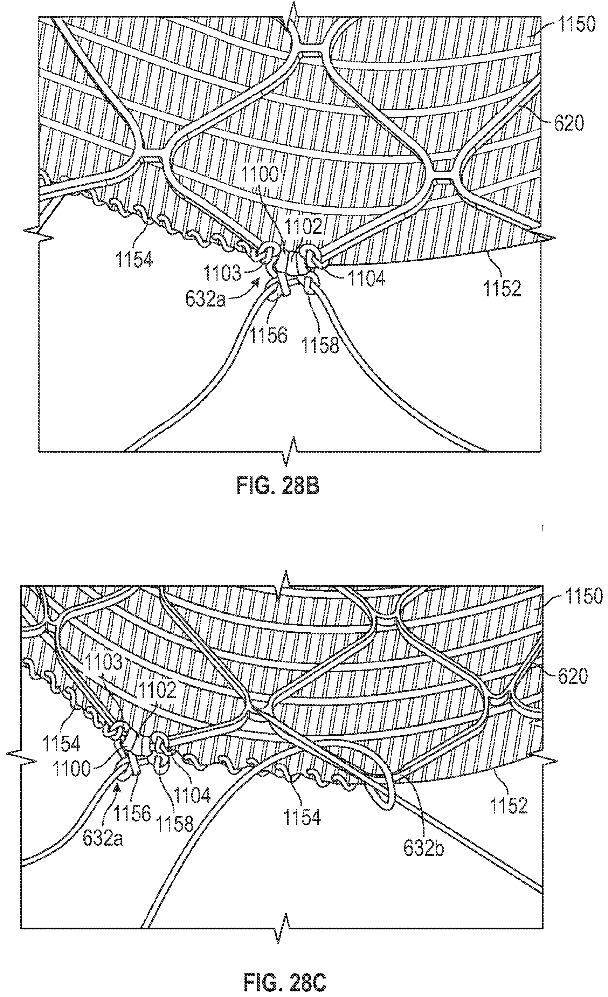

After forming the loops 1102, the last loop 1104 can be formed, in some examples by sewing one locking stitch with the covering element 1100 (FIG. 28B). In some examples, a locking stitch can be formed with the covering element 1100 around the first loop 1103, thereby forming a first knot 1156 and then another locking stitch can be formed around the last loop 1104, thereby forming a second knot 1158 (FIG. 28B). However, in other examples, only one knot or no knots can be formed adjacent to the loops 1102.

Whip stitches 1154 continuing from the first apex region 632a can then be formed around and through the inflow edge portion 1152 of the outer skirt 1150, between the first apex region 632a and an adjacent, second apex region 632b (FIG. 28C). This process can then be repeated to form the loops 1102 over each apex region 632 and whip stitches 1154 along the inflow edge portion 1152 of the outer skirt 1150, between adjacent apex regions 632.

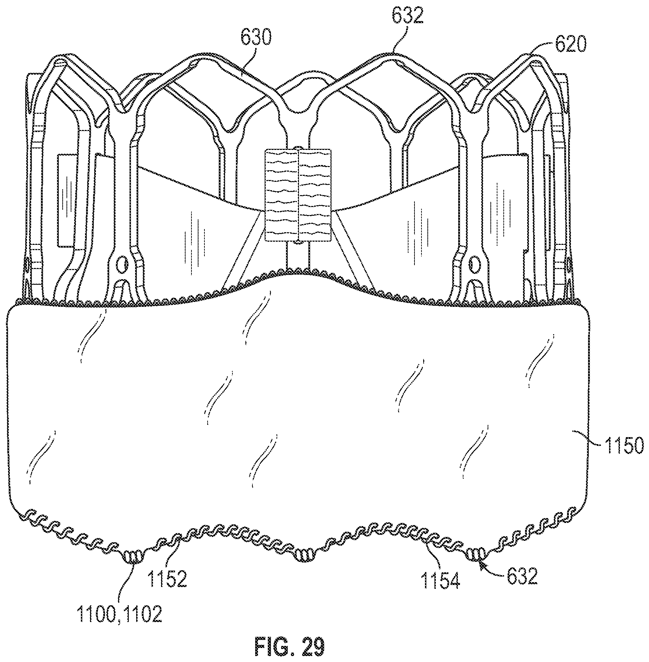

The final configuration of the prosthetic valve, including outer skirt 1150 secured to the frame 620 and the apex regions 632 covered by the covering element 1100, is shown in FIG. 29. It should be noted that though three loops 1102 of the covering element 1100 are shown extending though the outer skirt 1150 in FIG. 29, in alternate instances, fewer than three loops 1102 may extend though the outer skirt 1150 (e.g., two or only one). For example, in such instances, only a portion of the loops 1102 wrapping around the apex regions 632 may also extend through the outer skirt 1150. Further, the techniques described above for securing the outer skirt to an inflow end of the frame using a covering element that wraps around the apex regions (or apices) of the frame can be applied to a variety of different outer skirts and frames, such as any combination of the outer skirts and frames described herein.

By utilizing the same component (the covering element 1100) to both cover the apex regions of the frame and attach the outer skirt 1150 to the frame, the prosthetic heart valve can be assembled more quickly and easily. Additionally, by utilizing the same covering element 1100 (e.g., suture) for covering the inflow apex regions and securing the outer skirt 1150 to the frame, a crimp profile of the prosthetic heart valve can be reduced (as compared to using a first covering element or suture for covering the apex regions and a second element or suture to attach the outer skirt 1150 to the frame). Further still, by extending the covering element 1100 through the outer skirt 1150, the loops 1102 of the covering element 1100 can be more securely fastened in place around the apex regions such that they do not move or slip away from the apex regions during radial compression of the prosthetic heart valve. As a result, the covering element 1100 can remain covering the apex regions while the prosthetic heart valve is in the radially compressed configuration.

FIGS. 21-24 show additional examples of covering elements for one or more apex regions 502 of the frame 500 (or any of the other apex regions or apices of the frames described herein). As shown in FIGS. 21 and 22, a covering element 1200 can comprise a plurality of loops 1202 that wrap around one or more apex regions 502 at one end (e.g., the inflow end 508) of the frame 500. Though FIGS. 21 and 22 show the loops 1202 of the covering element 1200 covering an entirety of the inflow strut 562, in other examples, the loops 1202 can cover only the apex regions 502 (between the shoulders 540), as shown in FIG. 20. Further, in some examples, the covering element 1200 can comprise bridge portions that are the same or similar to the bridge portions 1106 shown in FIG. 20.

In some examples, as shown in FIGS. 21 and 22, the covering element 1200 can comprise knots 1204 formed between the loops 1202 covering adjacent inflow struts 562 (and/or outflow struts 560), the knots formed around strut junctions 510 between the adjacent inflow struts 562.

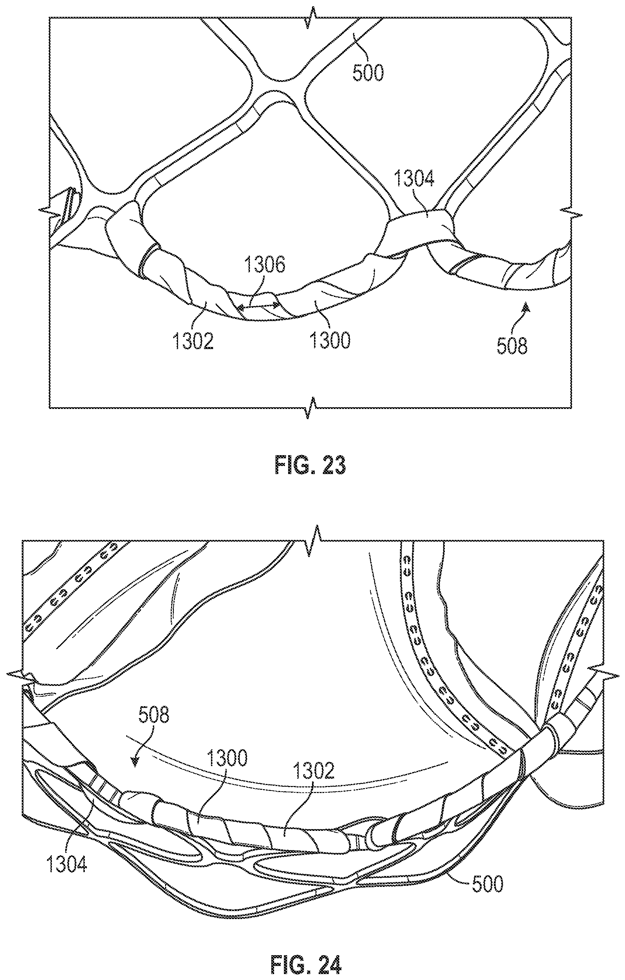

In alternate examples, the knots 1204 can be replaced by forming longitudinal slits in the strip or strand of the covering element 1200 and threading the covering element 1200 through itself at the position of the knots 1204 in FIGS. 21 and 22. For example, FIGS. 23 and 24 show such an instance where a covering element 1300 comprises a plurality of loops 1302 that wrap around the apex regions 502 at one end (e.g., the inflow end 508) of the frame 500 and a plurality of connecting portions 1304 that extend between adjacent inflow struts 562, across a strut junction 510 between the adjacent inflow struts 562.

As introduced above with reference to the covering element 1100 of FIG. 20, the covering element 1200 of FIGS. 21 and 22 and/or the covering element 1300 of FIGS. 23 and 24 can comprise a strip, strand, or suture comprising a material with a relatively low coefficient of friction, such as PTFE, UHMWPE, or PEEK, thereby facilitating smooth sliding of the prosthetic heart valve's inflow end 508 over and through the inner wall of the delivery sheath. For example, the coefficient between the covering element 1100 and the delivery sheath should be lower than the friction coefficient between a bare metal of the frame of the prosthetic heart valve and the delivery sheath.

In some examples, the covering elements described herein can have a static coefficient of friction in a range of 0.04 - 0.4, 0.04 - 0.2, or less than 0.2 relative to the inner wall of the delivery sheath. In some examples, the covering elements described herein can have a dynamic coefficient of friction in a range of 0.04 - 0.2, or 0.04 - 0.1, or less than 0.1 relative to the inner wall of the delivery sheath.

A width of the strip or band or suture that comprises the covering element 1100, 1200, and/or 1300 can be selected such that it enlarges the contact area of the apex regions 502 (e.g., at the inflow end 508) with the inner wall of the delivery sheath, and thereby reduces the shear stress to a value that is below the sheath yield strength resulting from the force (e.g., the axial force component) divided by the total contact area. This can, for example, reduce the likelihood of the valve snagging on the delivery apparatus (e.g., the sheath).

For example, FIGS. 21 and 22 show the covering element 1200 comprising a strip, band, or suture having a first width 1206, and FIGS. 23 and 24 show the covering element 1300 comprising a strip, band, or suture having a second width 1306, which is greater than the first width 1206.

In addition to reducing the shear stress of the apex regions 502 against the delivery sheath during navigating the prosthetic heart valve through the delivery sheath to the implantation site, the proposed covering elements can, for example, improve alignment between the prosthetic heart valve and the balloon on the delivery apparatus.

For example, during off-balloon crimping (e.g., radially compressing the prosthetic heart valve around the delivery apparatus, off of the inflatable balloon), the prosthetic heart valve can be disposed in a radially compressed configuration proximal to the deflated balloon, which can comprise a series of folds disposed around its circumference.

In practice, the folds of the balloon may not necessarily be folded in a neat or organized manner. Thus, when the radially compressed prosthetic heart valve is advanced toward and over the balloon upon reaching the implantation site, some folds of the balloon can catch on the strut junctions (e.g., strut junctions 510) at the inflow end of the frame and prevent further advancement of the prosthetic heart valve over the balloon. In some instances, this may even cause degradation to the balloon.

The bridge portions 1106 of the covering element 1100 (FIG. 20) can prevent such blocking of advancement of the prosthetic heart valve over the balloon from occurring. For example, the bridge portions 1106 can act as a barrier to the folds of the balloon, thereby blocking the balloon folds from catching on the strut junctions 510 at the inflow end. More specifically, as the prosthetic heart valve is being slid over top of the balloon, the bridge portions 1106 can contact the folds of the balloon and deflect these folds radially inward relative to the strut junctions 510 such that the valve can slide over the balloon in a relatively smooth manner.

Additionally, the smoother material of the covering element 1100, at the leading edge (e.g., inflow end) of the prosthetic heart valve can further facilitate proper alignment of the prosthetic heart valve over the balloon and/or reduced resistance.

Instead of or in addition to the covering element, the apex regions or apices of the frame (such as the apex regions at the inflow end of the frame) can be dip-coated or over-molded in a polymer so as to form a round-shaped cover over and/or around the apex regions or apices. Since polymeric materials can be softer and more flexible than most metals, such dip-coated or over-molded coverings may not impact the radial compression or expansion of the frame.

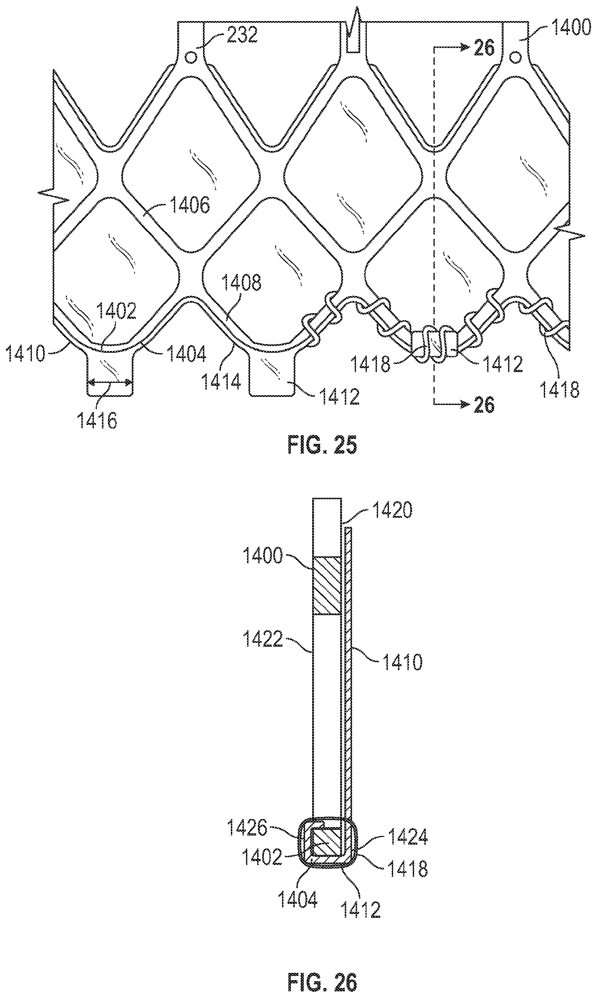

FIGS. 25 and 26 show another example of a covering element for apices or apex regions of a frame 1400 of a prosthetic heart valve. More specifically, FIGS. 25 and 26 present an example where the covering element is configured as a flap of a skirt 1410 (or perivalvular sealing member) of the prosthetic heart valve which is adapted to wrap around and cover a corresponding apex or apex region 1402 of the frame 1400. The frame 1400 can comprise a plurality of interconnected and angled struts 1406 and a plurality of apex regions 1402 at an inflow (or first) end 1404 of the frame 1400. The frame 1400 can be any of the frames described herein, such as one of frame 300 of FIG. 5, frame 400 of FIGS. 9A-9C, or frame 500 of FIGS. 10A-10C.

In some examples, a skirt 1410 (e.g., a fabric skirt) can be arranged around a surface of the frame 1400 and can comprise flaps 1412 that extend distally from a first edge 1414 of the skirt 1410. As shown in FIGS. 25 and 26, the first edge 1414 of the skirt 1410 can be an inflow edge that is attached to the inflow end 1404 of the frame 1400. The flaps 1412 of the skirt 1410 can be spaced apart from one another around a circumference of the skirt 1410.

In some examples, the skirt 1410 can be an annular skirt. In some examples, the skirt 1410 can comprise one or more skirt portions that are connected together and/or individually connected to the frame 1400. The skirt 1410 can comprise a fabric or polymeric material, such as ePTFE, PTFE, PET, TPU, UHMWPE, PEEK, PE, etc.

In some examples, the skirt 1410 can comprise a plurality of flaps 1412 which can include one flap 1412 for each apex region 1402 of the inflow end 1404 of the frame 1400.

Each flap 1412 can be configured to cover a corresponding apex region 1402. For example, each flap 1412 can have a width 1416 that is selected such that it covers at least a portion of, or in some instances an entirety of, the apex region 1402. However, in some examples, the width 1416 can be selected such that it does not cover an entire inflow strut 562 that the apex region 1402 is part of.

Further, the plurality of flaps 1412 can extend from the first edge 1414 of the skirt 1410 and be arranged along the first edge 1414 such that each flap 1412 is aligned with a corresponding apex region 1402 when the skirt 1410 is secured to the struts of the frame 1400 (as shown in FIGS. 25). As a result, each flap 1412 can be folded over the corresponding apex region 1402 and the inflow end 1404 of the frame 1400 and secured thereto by one or more stitches 1408 (or other fasteners, ultrasonic welding, and/or other means for securing), thereby covering the apex region 1402 in a manner that conceals the apex region 1402 and protects the inner wall of the delivery sheath from being contacted by the apex regions 1402 during navigation of the prosthetic heart valve through the delivery sheath to the implantation site.

In some examples, as shown in FIGS. 25 and 26, the skirt 1410 can be an inner skirt arranged around an inner surface 1420 (radially inward facing relative to a central longitudinal axis of the frame 1400) of the frame 1400. As such, the flaps 1412 can wrap around from the inner surface 1420, underneath the corresponding apex regions 1402 (at the inflow end 1404) and around to an outer surface 1422 of the frame 1400 (FIG. 26). The stitches 1418 (one shown in FIG. 26) can extend around the apex region 1402, through a first portion 1424 of the flap 1412 disposed over the inner surface 1420 of the apex region 1402, and around and/or through a second portion 1426 of the flap 1412 disposed over the outer surface 1422 of the apex region 1402 (FIG. 26). For example, in FIG. 26, the stitch 1418 is shown looping around the second portion 1426 of the flap 1412. However, in alternate examples, the stitch 1418 can additionally or alternately extend through the second portion 1426 of the flap 1412.

In other examples, the skirt 1410 can be an outer skirt arranged around the outer surface 1422 of the frame 1400. As such, the flaps 1412 can wrap around from the outer surface 1422, underneath the corresponding apex regions 1402 (at the inflow end 1404) and around to the inner surface 1420 of the frame 1400.

While the skirt 1410 is shown in FIGS. 25 and 26 as covering the narrower apex regions 1402 of the frame 1400, in other examples, the flaps 1412 of the skirt 1410 can be configured to wrap around and cover any type of inflow apex or apex region of a frame of a prosthetic heart valve (e.g., a squarer or pointed apex, such as the apex 220 of frame 200 in FIG. 3).

It should be noted that for the purpose of illustration, FIG. 26 is schematic and thus small gaps are shown between the components (e.g., the frame 1400 and skirt 1410 and/or the skirt 1410 and the apex region 1402) in order to distinguish the different components more easily. However, in some examples, these gaps may be smaller than shown or there may be little to no gaps between certain components (e.g., the stitch 1418 and flap 1412). Further, while FIG. 26 depicts the flap 1412 as having more square corners (due to wrapping), in some examples the flap 1412 can have a more rounded and flexible wrapped shape (e.g., due to it comprising a fabric or polymeric material).

A skirt comprising flaps configured to cover apices or apex regions at an end of the frame of a prosthetic heart valve (e.g., a leading end of the valve during advancing the radially compressed prosthetic heart valve through the delivery sheath to an implantation site, which in some examples can be the inflow end) can prevent the apices or apex regions from penetrating and/or damaging the inner wall of the delivery sheath, as well as reduce the push force required for advancement of the prosthetic heart valve through the delivery sheath with the delivery apparatus.

Further details and examples for cushioning or covering elements configured to cover at least a portion of one or more apices of a frame of a prosthetic heart valve can be found in International Patent Application No. PCT/US2020/044994, filed August 5, 2020, and which claims the benefit of U.S. Provisional Application Ser. No. 62/886,677, filed August 14, 2019.

As described above, a frame for a prosthetic heart valve comprising apex regions with a reduced width, which extends for a length that is at least 25% of a length of the inflow or outflow strut comprising the apex region, can decrease maximal stresses experienced by the frame during expansion of the frame and move the maximal stresses away from the apex regions, thereby increasing a robustness and longevity of the frame. Further, apex regions with a single, continuous curve that curves between corresponding angled strut portions of the inflow or outflow strut and that form an angle between 120 degrees and 140 degrees between the two angled strut portions can result in an apex region with a minimal axial height (e.g., which is equal to the width of the apex region), thereby enabling cusp edge portions of leaflets of the prosthetic heart valve to be arranged closer to the inflow end of the frame and an axial height of a first row of cells disposed at the outflow end of the frame to be increased, thereby providing more open space (not blocked by the commissures and outflow edges of the leaflets) at the outflow end of the frame for increased blood flow and coronary access. Additionally, frames comprising apex regions defining this larger angle (e.g., greater than 120 degrees and up to 140 degrees) can have reduced radial recoil after deflating the balloon of the delivery apparatus, after radially expanding the prosthetic heart valve. Further still, such apex regions can be more atraumatic and interact less with the balloon of the delivery apparatus and/or a delivery sheath, when the prosthetic heart valve is mounted on the delivery apparatus and navigated to a target implantation site.

In another example, the strain concentrations and/or maximal stresses experienced at the apices of frames with reduced height apices or apex regions having thinned regions (such as those shown in FIGS. 5 and 6) can be reduced by creating a bump or protrusion at a central portion (or center) of the apex. As described in further detail below, this bump can, for example, reinforce the apical center of the apex and distribute strains between the thinner regions of the apex on both sides of the bump, thereby reducing the likelihood of material degradation due to high strains experienced during bending at the apex during expansion of the frame.

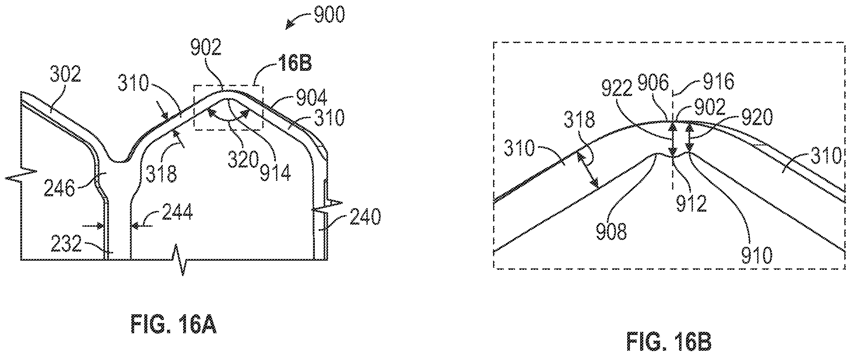

FIGS. 16A and 16B show an exemplary example of a portion of a frame 900 for a prosthetic heart valve, where the frame 900 can be similar to the frame 300 of FIGS. 5 and 6, except for the configuration of the apices. For example, the frame 900 can comprise interconnected struts 302 forming apices (or apex regions) 902 at an inflow end and outflow end 904 (shown in FIGS. 16A and 16B) of the frame 900. Similar to the frame 300 of FIGS. 5 and 6, each apex 902 is disposed between and forms a transition between two angled struts 310 at the inflow end or outflow end 904 (FIGS. 16A and 16B) of the frame 900. A detail view of a single apex 902 is shown in FIG. 16B.

Each apex 902 can include a curved or relatively flat outer surface 906 (axially facing toward the outflow end 904) and an arcuate or curved inner surface 914 disposed opposite the outer surface 906 (axially facing toward the inflow end). In contrast to the single inner depression of apices 304 of FIGS. 5 and 6, the inner surface 914 (FIG. 16A) of each apex 902 comprises two curved inner depressions, including a first inner depression 908 and a second inner depression 910, separated from one another by a bump 912 (FIG. 16B). In this manner, the inner surface 914 of the strut at the apex 902 generally comprises an "M" shape.

As shown in FIG. 16B, the bump 912 can protrude axially away from the first inner depression 908 and the second inner depression 910. Further, the bump 912 can be disposed at a central region or center of the apex 902, as denoted in FIG. 16B by central longitudinal axis 916. For example, the peak of the apex 902 and the peak of the bump 912 (which extend in opposite axial directions) can be aligned along the central longitudinal axis 916.

The first inner depression 908 and the second inner depression 910 can create thinned regions of the apex 902 (on either side of the bump 912) having a width (or height) 920 that is smaller than a width 318 of the angled struts 310 (FIG. 16B). In some examples, the width 920 can be the same or similar to the width 316 of the inner depression 314 of the apex 304 of the frame 300 (FIG. 6). However, a region of the apex 902 at the bump 912 can have a width 922 that is larger than the width 920 of the first inner depression 908 and the second inner depression 910 (FIG. 16B). In some examples, the width 922 is still smaller than the width 318 of the angled struts 310.

In some examples, a difference between the width 922 and the width 920, which can be referred to as a height of the bump 912, can be on the order of microns (micrometers), and thus, not visible with the naked eye. The bump 912 shown in FIG. 16B is exaggerated slightly for the purposes of illustration (and in actuality may be smaller than it appears in FIG. 16B).

In some examples, the first inner depression 908 and the second inner depression 910 and the bump 912 can be formed with a laser. In some examples, the bump 912 can be within the dimensional tolerance of the strut (±0.025 mm) and can be formed by increasing the resolution of the laser beam at the apical regions (e.g., apex 902) in which the bump 912 is formed. In addition to the regular ±0.025 mm tolerance, a local tolerance referring to the width difference between the width 922 and width 920, thus locally following the cut curve, is provided. This tolerance can, in some instances, be about an order of magnitude tighter than the regular tolerance (e.g., 0.0025 mm, or in other instances 1-10 µm). Thus, in some examples, the width difference between the width 922 and the width 920, and thus the height of the bump 912, can be 25 µm ± 2.5 µm. In some examples, the height of the bump 912 can be in a range of 10 to 50 µm or 20 to 30 µm.

In some examples, since the struts of the frame 900 can have a substantially rectangular cross-sectional shape, for which the moment of inertia is I=(1/12)bh³ (where "h" refers to a height in the axial direction, which can be the same as the width referred to above, and "b" refers to a thickness in a radial direction), the stress developed during bending of the apex 902 is proportional to the bending moment divided by the moment of inertia. Thus, a small increase in the height (or width) of the strut substantially influences (by a third power) stress/strain developed at the apex 902 (or other regions of the struts).

Thus, the shape of the bump 912, which results in an increased width 922 (or height) at the center of the apex 902, reduces the strains developed at the central region of the apex 902 while maximal strains are spread to both neighboring thinned regions of the apex 902 on either side of the bump 912 (at the first inner depression 908 and the second inner depression 910). This, in turn, can significantly reduce the strains at the reduced height apex 902, thereby allowing the apex 902 to withstand bending forces (e.g., during radial expansion of the frame 900) without experiencing material degradation.

As explained above, in some examples, a frame of a prosthetic heart valve can comprise axial struts (e.g., axial struts 232) that have an increased width relative to widths of the angled struts of the frame (including the angled struts to which the axial strut is connected and extends between). As a result, a larger contact area is provided for when the leaflets of the prosthetic heart valve contact the wider axial struts during systole, thereby distributing the stress and reducing the extent to which the leaflets may fold over the axial struts, radially outward through the cells of the frame. Thus, as one example, a long-term durability of the leaflets of the prosthetic heart valve can be increased.

Widened axial struts may, in some instances, cause coronary access problems following a valve-in-valve procedure. For example, in some situations, a second prosthetic heart valve can be implanted within a previously implanted, first prosthetic heart valve. After such a valve-in-valve implantation procedure, the axial struts of the first and second prosthetic heart valves may be positioned in close proximity (e.g., adjacent) to each other. If coronary access is required with a coronary access catheter and access is blocked by the adjacent axial struts of the first and second prosthetic heart valves, a balloon catheter can usually be advanced between the adjacent axial struts of the first and second prosthetic heart valves and inflated to expand or bend the axial struts sideways and out of the way, thereby creating a larger opening for coronary access. However, the widened axial struts disclosed herein may result in increased resistance to the inflated balloon, and in some cases, the adjacent axial struts may not be able to be separated for coronary access.

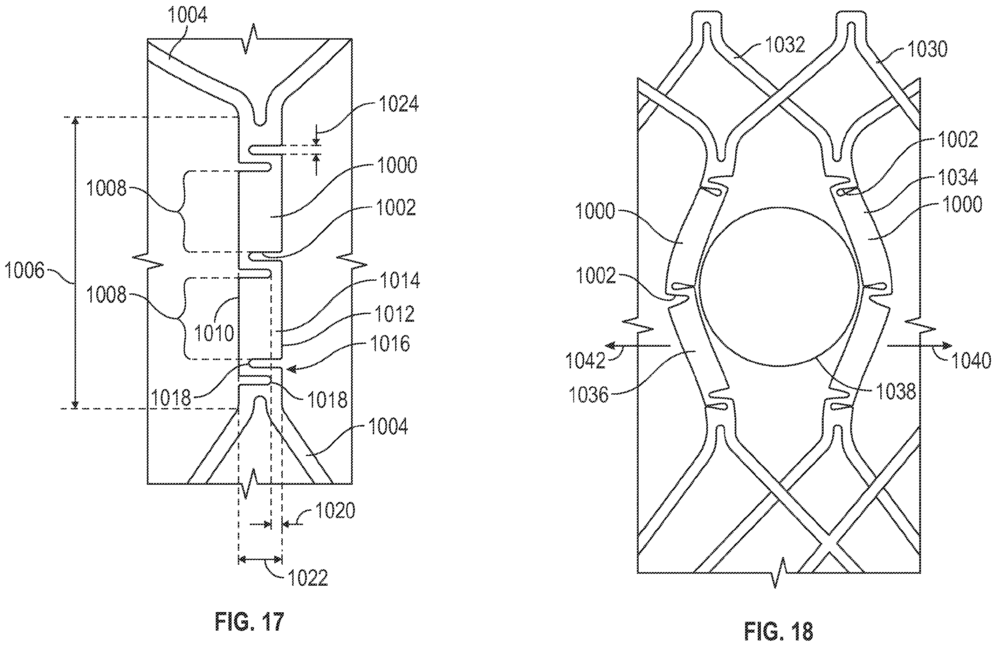

To improve coronary access, a prosthetic heart valve with wider axial struts can also include a plurality of lateral slits (or notches) in the axial struts, along an axial length of the axial struts. An exemplary widened axial strut 1000 having a lateral width 1022 and comprising one or more slits 1002 is shown in FIG. 17. In some examples, as shown in FIG. 17, the one or more slits 1002 include a plurality of slits 1002. As described above, in some examples, the width 1022 of the axial strut 1000 can be in a range of 0.45 mm - 1.0 mm, 0.5 mm - 0.75 mm, or at least 0.6 mm.

The axial strut 1000 can be coupled to and extend between angled struts 1004 of the prosthetic heart valve. An axial length 1006 of the axial strut 1000 can be defined between a point where lower ends of two angled struts 1004 converge and a point where upper ends of another two angled struts 1004 converge. The slits 1002 can be spaced apart from each other along the axial length 1006 of the axial strut 1000.

**In** some examples, the slits 1002 can be grouped into groups (e.g., pairs) 1016 of slits 1002 that are spaced apart from one another along the axial length 1006, with smaller amounts of space (in an axial direction) between the slits 1002 included in a same group, thereby creating one or more solid or slit-free portions 1008 in the axial strut 1000 (e.g., portions that do not contain slits 1002). Said another way, each group 1016 can be spaced apart from an adjacent group 1016, along the length 1006 of the axial strut 1000, by an amount or spacing that is greater than a spacing between slits 1002 within the same group 1016.

**In** other examples, the plurality of slits 1002 of the axial strut 1000 can be spaced equally apart from one another along the axial length 1006.

**In** some examples, the axial spacing or distance between slits 1002 within the same group 1016 can vary (e.g., can be smaller or larger than shown in FIG. 17).

**In** some examples, the axial strut 1000 can comprise a first lateral edge 1010, a second lateral edge 1012, and a radially inward facing surface 1014 extending between the first lateral edge 1010 and the second lateral edge 1012. Each slit 1002 can extend, in a lateral direction, from one of the first lateral edge 1010 and second lateral edge 1012, in a lateral (or circumferential) direction into the axial strut 1000, and toward the other one of the first lateral edge 1010 and second lateral edge 1012. **In** this way, each slit 1002 can extend through a portion of the width 1022 of the axial strut 1000 (e.g., only the portion and not through the entire width 1022).

For example, a first portion of slits 1002 (e.g., one for each pair or group 1016) can extend into the axial strut 1000 from the first lateral edge 1010 toward the second lateral edge 1012, with a closed end 1018 of the slit 1002 spaced away from the second lateral edge 1012 by a (lateral) distance 1020. Likewise, a second portion of slits 1002 (e.g., one for each pair or group 1016) can extend into the axial strut 1000 from the second lateral edge 1012 toward the first lateral edge 1010, with the closed end 1018 of the slit 1002 spaced away from the second lateral edge 1012 by the (lateral) distance 1020. In some examples, the distance 1020 can be in a range of 0.1 - 0.3 mm and the width 1022 can be in a range of 0.5 - 1.0 mm.

In some examples, the distance 1020, and thus a width (in the lateral direction) of each slit 1002 can vary for slits in the same axial strut 1000 and/or for different axial struts. The width of a slit 1002 can be the difference between the width 1022 of the axial strut 1000 and the distance 1020. As example, in one instance, a first slit 1002 can have a first width and a second slit 1002 of the same axial strut 1000 can have a second width, the second width smaller than the first width (and thus, the distance 1020 would be greater for the second slit than the first slit). In other examples, the width of each slit 1002 in the axial strut 1000 can be the same (and thus, the distance 1020 would be the same for each slit 1002).

Each slit 1002 can have an axial height 1024 in a range of 0.075 - 0.3 mm. In some examples, all slits 1002 in the axial strut 1000 can have the same height 1024. In other examples, one or more slits 1002 of the plurality of slits in the axial strut 1000 can have a different height (smaller or larger) than other slits 1002 of the plurality of slits in the axial strut 1000.

In some examples, the slits 1002 are formed in the axial strut 1000 with a laser (e.g., by laser cutting).

The slits 1002 can be referred to as release slits and can be configured to increase a compliance (e.g., flexibility) of the axial strut 1000 such that when a balloon of a balloon catheter inserted between adjacent axial struts of two (concentrically implanted) prosthetic heart valves is inflated between the adjacent axial struts, the adjacent axial struts bend laterally outward and away from one another, thereby creating a space therebetween for coronary access. For example, as shown in FIG. 18, during a valve-in-valve procedure, a second (guest) prosthetic heart valve 1030 can be inserted, radially expanded, and implanted within an originally implanted, first (host) prosthetic heart valve 1032. A first axial strut 1034 of the first prosthetic heart valve 1032 can be disposed proximate and adjacent to a second axial strut 1036 of the second prosthetic heart valve 1030 (both the first axial strut 1034 and the second axial strut 1036 being the same as axial strut 1000).

If the first axial strut 1034 and the second axial strut 1036 are blocking coronary access (e.g., due to their positioning in front of an opening to a coronary artery and their close proximity to one another), a balloon catheter can be positioned between the first axial strut 1034 and the second axial strut 1036 and then a balloon 1038 of the balloon catheter can be inflated. As the balloon 1038 is inflated and applies pressure against the lateral edges of the first axial strut 1034 and the second axial strut 1036, the first axial strut 1034 and the second axial strut 1036 are bent outward, in opposite directions, due to their slits 1002. As such, the first axial strut 1034 and the second axial strut 1036 are bent away from one another (or buckle), creating a space for coronary access between the first axial strut 1034 and the second axial strut 1036.

By having slits 1002 that extend from both lateral edges (sides) of the axial strut 1000, the axial strut 1000 can bend in any of two (lateral or circumferential) directions, depending on which side of the axial strut 1000 the balloon is positioned. For example, as shown in FIG. 18, the first axial strut 1034 bends in a first direction 1040 and the second axial strut 1036 bends in an opposite, second direction 1042.

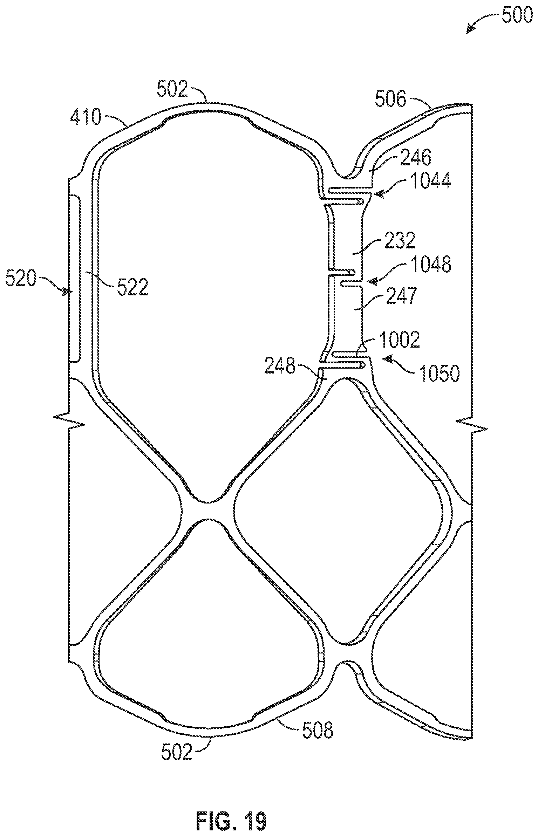

The axial struts of any of the prosthetic heart valves described herein can be configured the same or similarly to the axial struts 1000 of FIGS. 17 and 18. For example, as shown in FIG. 19, the axial strut 232 of frame 500 (FIGS. 10A-12) can comprise the plurality of slits 1002. **In** some examples, as shown in FIG. 19 a first group of slits 1044 can be disposed in the wider outflow end portion 246 of the axial strut 232, a second group of slits 1048 can be disposed in the narrower middle portion 247 of the axial strut 232, and a third group of slits 1050 can be disposed in the wider inflow end portion 248 of the axial strut 232.

**In** some examples, as shown in FIG. 19, a width (in the lateral direction) of the slits 1002 in the first group of slits 1044 and the third group of slits 1050 can be larger than a width of the slits 1002 in the second group of slits 1048. As a result, the distance (e.g., distance 1020 shown in FIG. 17) between the closed end of the slit 1002 and the lateral edge of the axial strut can be the same or similar for all slits 1002. **In** other examples, a width of the slits 1002 can be the same for all the groups 1044, 1048, and 1050.

In other examples, all the slits 1002 in the axial strut 232 can be disposed in the middle portion 247, without any slits in the wider outflow and inflow end portions 246, 248.

Though the examples of FIGS. 17-19 show six slits 1002 in each axial strut, in other examples, each axial strut can comprise more or less than six slits 1002 (e.g., four, five, eight, or the like). In some examples, each axial strut can only include one slit 1002.

However, in some examples, three groups of two slits 1002 (six slits 1002 in total, as shown in the examples of FIGS. 17-19), where the two slits of the same group extend in opposite lateral directions, can be advantageous. For example, such a configuration provides three bending points in each direction (lateral direction), thereby enabling the axial strut 1000 to bend in either of two lateral directions (e.g., depending on which side of the axial strut a lateral force from an inflating balloon is applied).

Additionally, in some examples, the slits 1002 can have different shapes than those shown in FIGS. 17-19, such as triangular, square, tapered, or the like (in contrast to oblong or rectangular with a curved closed end). Further, as mentioned above, a width and height of the slits 1002 can vary.

As introduced above, frames can have axial struts that are wider than the angled struts of the frame to which they are connected. The axial struts can include non-commissure window axial struts (e.g., axial struts 232) and axially extending struts (comprising axially extending window strut portions, such as axially extending window strut portions 240) that define commissure windows therein. Regions of higher stress can be formed at the transition between the narrower angled struts and wider axial struts (or axially extending window strut portions). In particular, the higher stress regions can occur at a root or base of an angled strut at the outflow end of the frame, where the root of the angles strut curves outward (in a convex fashion) to a larger width of the axial strut. These higher stress regions can be exacerbated at the commissure window axial struts due to these struts experiencing higher loads during operating of the prosthetic heart valve (e.g., since the leaflet commissure tabs are attached directly to the commissure windows defined by the axially extending window strut portions of the axial struts). Thus, it is desirable to strengthen the frame in these regions such that a durability of the frame is increased.

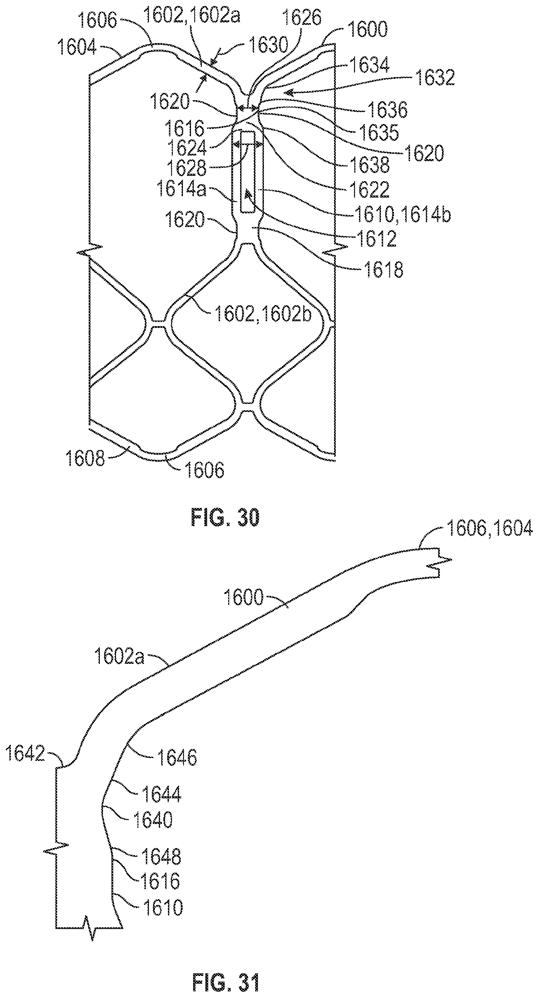

As one example, the stresses experienced in these transition regions can be reduced, thereby strengthening the frame, by adding a concave region (or depression that can result in a narrowed region) at an end portion of the axially extending window strut portions and/or axial struts, where they connect directly to a root or base of the angled struts of the frame. Examples of such concave regions applied to an exemplary frame 1600 for a prosthetic heart valve are shown in FIGS. 30 and 31. Though FIGS. 30 and 31 depict frame 1600, which can be similar to the frame 400 or frame 500 described above, the concave regions described below with reference to FIGS. 30 and 31 can be applied to a variety of prosthetic heart valve frames, including any of the frames described herein.

Turning first to FIG. 30, the frame 1600 can comprise a plurality of rows of angled struts 1602, including a first row of angled struts 1602a that form an outflow end 1604 of the frame 1600. The frame 1600 can include apex regions 1606 (or in other examples apices, such as those shown in FIGS. 1, 3, and 5) formed at the outflow end 1604 and an inflow end 1608 of the frame 1600. The frame 1600 can also include a plurality of axial struts 1610 (one shown in FIG. 30) defining a commissure window 1612 therein. For example, each axial strut 1610 can comprise a first window strut portion 1614a and a second window strut portion 1614b defining the commissure window 1612 and forming an upper (outflow) end portion 1616 above (or further toward the outflow end 1604) the commissure window 1612 and a lower (or inflow) end portion 1618 below (or further toward the inflow end 1608) the commissure window 1612. The lower end portion 1618 can connect to a second row of angled struts 1602b and the upper end portion 1616 can connect to the angled struts 1602a.

In some examples, as shown in FIG. 30, a concave region 1620 (which can form or continue along the axial strut as a thinned or narrowed region) can be formed in the upper end portion 1616, at a root or base of the angled strut 1602a to which it connects. In particular, a transition region 1632 between the angled strut 1602a and the axial strut 1610 can include three changes in concavity that result in lower stresses at the base of the angled strut 1602a, where the angled strut 1602a connects to the upper portion 1616 of the axial strut 1610. The three changes in concavity includes a first concave curve 1634 (or concave region), which transitions to a first convex curve 1636 (or convex region), and then a second concave curve 1635 which forms the concave region 1620. A fourth change in concavity can occur at the base of the upper portion 1616, as shown by the second convex curve 1638 which transitions back to the wider portion of the axial strut 1610. Thus, a more gradual transition can be created between the narrower angled struts 1602a and a wider portion 1624 (having a wider width 1628) of the axial strut 1610. As a result, maximal stresses experienced at the transition regions 1632, or at the base or root of the angled struts 1602a can be reduced, thereby increasing a durability of the frame 1600.

**In** some instances, each upper end portion 1616 of each axial strut 1610 can include two concave regions 1620 (or concavities), one on each side of the upper end portion 1616, at the base of the angled strut 1602a. As a result, narrowed regions 1622 having a narrower width 1626 are created in the upper end portion 1616, adjacent to the angled struts 1602a. For example, the width 1626 of the narrowed regions 1622 can be larger than a width 1630 of the angled struts 1602a but smaller than the width 1628 of the wider portion 1624 of the axial strut 1610.

**In** some examples, the lower end portion 1618 can also include concave regions 1620 therein, thereby creating a more gradual transition between the wider portion 1624 of the axial strut 1601 and the angled struts 1602b.

FIG. 31 shows another example of a concave region 1640 that can be formed in the upper end portion 1616, at a base or root of the angled strut 1602a. The concave region 1640 can be formed as a circumferentially extending bite or indent into the upper end portion 1616, at the base of the angled strut 1602a. **In** some instances, as shown in FIG. 31, the concave region 1640 can be formed immediately adjacent to a convex curve 1644 in the base of the angled strut 1602a which is immediately adjacent to another concave curve 1646 in the angled strut 1602a. Additionally, in some instances, the concave region 1640 can transition to another convex curve 1648 in the axial strut 1610.

The at least three changes in concavity between the concave curve 1646, the convex curve 1644, and the concave region 1640 (and optionally the 4^{th} change in concavity at the concave curve 1646) can result in reduced stresses at the base of the angled strut 1602a, where it connects to the axial strut 1610. Though only one side of the axial strut is shown in FIG. 31, in some examples, both sides of the upper end portion 1616 can include a concave region 1640. Further, in some instances, the lower end portion 1618 of the axial strut 1610 can also include the concave regions 1640 at the base of the angled struts 1602b (similar to as shown in FIG. 30).

In some examples, the concave regions 1640 of FIG. 31 or the concave regions 1620 of FIG. 30 can be included on only the axial struts (at the base of the angled struts to which they are connected) defining the commissure windows (e.g., the axially extending window strut portions described herein).

In alternate examples, the concave regions 1640 of FIG. 31 or the concave regions 1620 of FIG. 30 can be included on all the axial struts of the frame, at the base of the angled struts to which they are connected, including the axial struts forming the commissure windows and the non-commissure window axial struts (e.g., axial struts 232).

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) are introduced into the aorta through a surgical incision in the ascending aorta, such as through a partial J-sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

**In** all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

## Claims

1. A prosthetic heart valve comprising:
a radially expandable and compressible annular frame (400; 500) comprising:
a plurality of interconnected struts defining a plurality of rows of cells arranged between an inflow end (408; 508) and an outflow end (406; 506) of the frame (400; 500), the plurality of interconnected struts comprising a plurality of outflow struts (460; 560) defining the outflow end (406; 506) and a plurality of inflow struts (462; 562) defining the inflow end (402, 404; 502), wherein each outflow strut (460; 560) comprises two angled strut portions (410; 510) interconnected by an apex region (402, 404; 502), and wherein each inflow strut (462; 562) comprises two angled strut portions (410; 510) interconnected by an apex region (404; 502),
wherein each apex region (402, 404; 502) curves between a corresponding pair of two angled strut portions (410; 510), wherein each apex region (402, 404; 502) has a narrowed width (416) compared to the angled strut portions, that extends along a length (425) of the outflow strut (460; 560) and inflow strut (462; 562), and wherein the narrowed width (416) is smaller than a width (418) of the two angled strut portions (410; 510).

2. The prosthetic heart valve of claim 1, wherein each apex region (402, 404; 502) forms an angle (440) between the two angled strut portions (410; 510) of a corresponding outflow strut (460; 560) and/or inflow strut (462; 562) that is greater than 120 degrees and up to 140 degrees.

3. The prosthetic heart valve of either claim 1 or claim 2, wherein each apex region (402, 404; 502) includes a curved outer surface (428) with a radius of curvature that is greater than 1 mm, the curved outer surface (428) extending between outer surfaces of the two angled strut portions (410; 510) of a corresponding outflow strut (460; 560) and/or inflow strut (462; 562).

4. The prosthetic heart valve of any one of claims 1 to 3, wherein the length of each apex region (402, 404; 502) is in a range of 0.9 mm to 2.2 mm.

5. The prosthetic heart valve of any one of claims 1 to 3, wherein the length of each apex region (402, 404; 502) is in a range of 1.9 mm to 2.2 mm.

6. The prosthetic heart valve of any one of claims 1 to 3, wherein the length of each apex region (402; 502) at the outflow end (406; 506) is in a range of 1.8 mm to 2.4 mm and wherein the length of each apex region (404; 502) at the inflow end (402; 502) is in a range of 0.8 mm to 1.2 mm.

7. The prosthetic heart valve of any one of claims 1 to 6, wherein the narrowed width (416) of each apex region (402, 404; 502) is from 0.06 mm to 0.15 mm smaller than the width (418) of the two angled strut portions (410; 510).

8. The prosthetic heart valve of any one of claims 1 to 7, further comprising a plurality of leaflets (204) secured to the frame (400; 500) and a plurality of commissure windows (520) formed by struts of the plurality of interconnected struts forming cells of a first row of cells (324) of the plurality of rows of cells, the first row of cells (324) disposed at the outflow end (406; 506) of the frame (400; 500), and wherein each commissure window (242, 520) is configured to receive commissure tabs of two adjacent leaflets (204) of the plurality of leaflets (204).

9. The prosthetic heart valve of claim 8, wherein each commissure window (520) is defined by axially extending window strut portions (514) that form an upper end portion (516) above the commissure window and a lower end portion (518) below the commissure window (520) and wherein a length (528), in an axial direction relative to a central longitudinal axis of the frame (400; 500), of the upper end portion (516) and the lower end portion (518) is larger than the width (1630) of the two angled strut portions (410).

10. The prosthetic heart valve of claim 9, wherein the upper end portion (1616) includes a concave region (1620) disposed therein, adjacent to a convex curve (1634) at a base of a first angled strut portion (1602a) of the two angled strut portions to which the upper end portion (1616) connects, and wherein the convex curve (1636) extends from a concave curve (1634) in the first angled strut portion (1602a).

11. The prosthetic heart valve of any one of claims 1 to 10, wherein a covering element (1100) is wrapped around and covering the apex region (402, 404; 502) of the frame (400; 500).

12. The prosthetic heart valve of claim 11, wherein the covering element (1100) comprises a plurality of consecutive loops (1102) of a strip or band of material.

13. The prosthetic heart valve of claim 12, wherein the plurality of consecutive loops (1102) of the strip or band of material are wrapped around the two thinned strut portions (414), between the shoulders of the apex region (402, 404; 502).

14. The prosthetic heart valve of any one of claims 11 to 13, further comprising an outer skirt (1150) disposed around an outer surface of the frame (400; 500), and wherein the covering element (1100) extends through the outer skirt (1150) such that the outer skirt (1150) is secured to the frame (400, 500).

15. The prosthetic heart valve of any one of the preceding claims, further comprising an inner skirt (1410) arranged on and/or coupled to an inner surface (1420) of the frame (400; 500).

## Patentansprüche

1. Herzklappenprothese, umfassend:
einen radial expandierbaren und komprimierbaren ringförmigen Rahmen (400; 500), umfassend:
eine Vielzahl von miteinander verbundenen Streben, die eine Vielzahl von Zellreihen definieren, die zwischen einem Einströmende (408; 508) und einem Ausströmende (406; 506) des Rahmens (400; 500) angeordnet sind, wobei die Vielzahl von miteinander verbundenen Streben eine Vielzahl von Ausströmstreben (460; 560), die das Ausströmende (406; 506) definieren, und eine Vielzahl von Einströmstreben (462; 562), die das Einströmende (402, 404; 502) definieren, umfasst, wobei jede Ausströmstrebe (460; 560) zwei angewinkelte Strebenabschnitte (410; 510) umfasst, die durch einen Spitzenbereich (402, 404; 502) miteinander verbunden sind, und wobei jede Einströmstrebe (462; 562) zwei angewinkelte Strebenabschnitte (410; 510) umfasst, die durch einen Spitzenbereich (404; 502) miteinander verbunden sind,
wobei sich jeder Spitzenbereich (402, 404; 502) zwischen einem entsprechenden Paar von zwei angewinkelten Strebenabschnitten (410; 510) krümmt, wobei jeder Spitzenbereich (402, 404; 502) im Vergleich zu den angewinkelten Strebenabschnitten eine verringerte Breite (416) aufweist, die sich entlang einer Länge (425) der Ausströmstrebe (460; 560) und der Einströmstrebe (462; 562) erstreckt, und wobei die verringerte Breite (416) kleiner ist als eine Breite (418) der zwei angewinkelten Strebenabschnitte (410; 510).

2. Herzklappenprothese nach Anspruch 1, wobei jeder Spitzenbereich (402, 404; 502) zwischen den zwei angewinkelten Strebenabschnitten (410; 510) einer entsprechenden Ausströmstrebe (460; 560) und/oder Einströmstrebe (462; 562) einen Winkel (440) bildet, der größer als 120 Grad und bis zu 140 Grad beträgt.

3. Herzklappenprothese nach Anspruch 1 oder Anspruch 2, wobei jeder Spitzenbereich (402, 404; 502) eine gekrümmte Außenfläche (428) mit einem Krümmungsradius aufweist, der größer als 1 mm ist, wobei sich die gekrümmte Außenfläche (428) zwischen Außenflächen der zwei angewinkelten Strebenabschnitte (410; 510) einer entsprechenden Ausströmstrebe (460; 560) und/oder Einströmstrebe (462; 562) erstreckt.

4. Herzklappenprothese nach einem der Ansprüche 1 bis 3, wobei die Länge jedes Spitzenbereichs (402, 404; 502) in einem Bereich von 0,9 mm bis 2,2 mm liegt.

5. Herzklappenprothese nach einem der Ansprüche 1 bis 3, wobei die Länge jedes Spitzenbereichs (402, 404; 502) in einem Bereich von 1,9 mm bis 2,2 mm liegt.

6. Herzklappenprothese nach einem der Ansprüche 1 bis 3, wobei die Länge jedes Spitzenbereichs (402; 502) am Ausströmende (406; 506) in einem Bereich von 1,8 mm bis 2,4 mm liegt und wobei die Länge jedes Spitzenbereichs (404; 502) am Einströmende (402; 502) in einem Bereich von 0,8 mm bis 1,2 mm liegt.

7. Herzklappenprothese nach einem der Ansprüche 1 bis 6, wobei die verringerte Breite (416) jedes Spitzenbereichs (402, 404; 502) um 0,06 mm bis 0,15 mm kleiner ist als die Breite (418) der zwei angewinkelten Strebenabschnitte (410; 510).

8. Herzklappenprothese nach einem der Ansprüche 1 bis 7, ferner umfassend eine Vielzahl von Klappensegeln (204), die an dem Rahmen (400; 500) befestigt sind, und eine Vielzahl von Kommissurfenstern (520), die durch Streben der Vielzahl von miteinander verbundenen Streben gebildet sind, die Zellen einer ersten Reihe von Zellen (324) der Vielzahl von Zellreihen bilden, wobei die erste Reihe von Zellen (324) an dem Ausströmende (406; 506) des Rahmens (400; 500) angeordnet ist, und wobei jedes Kommissurfenster (242, 520) dazu konfiguriert ist, Kommissurlaschen von zwei benachbarten Klappensegeln (204) der Vielzahl von Klappensegeln (204) aufzunehmen.

9. Herzklappenprothese nach Anspruch 8, wobei jedes Kommissurfenster (520) durch sich axial erstreckende Fensterstrebenabschnitte (514) definiert ist, die einen oberen Endabschnitt (516) oberhalb des Kommissurfensters und einen unteren Endabschnitt (518) unterhalb des Kommissurfensters (520) bilden, und wobei eine Länge (528) des oberen Endabschnitts (516) und des unteren Endabschnitts (518), in einer axialen Richtung bezogen auf eine zentrale Längsachse des Rahmens (400; 500), größer ist als die Breite (1630) der zwei angewinkelten Strebenabschnitte (410).

10. Herzklappenprothese nach Anspruch 9, wobei der obere Endabschnitt (1616) einen darin angeordneten konkaven Bereich (1620) aufweist, der benachbart zu einer konvexen Krümmung (1634) an einer Basis eines ersten angewinkelten Strebenabschnitts (1602a) der zwei angewinkelten Strebenabschnitte ist, mit dem der obere Endabschnitt (1616) verbunden ist, und wobei sich die konvexe Krümmung (1636) von einer konkaven Krümmung (1634) in dem ersten angewinkelten Strebenabschnitt (1602a) aus erstreckt.

11. Herzklappenprothese nach einem der Ansprüche 1 bis 10, wobei ein Abdeckelement (1100) um den Spitzenbereich (402, 404; 502) des Rahmens (400; 500) gewickelt ist und diesen bedeckt.

12. Herzklappenprothese nach Anspruch 11, wobei das Abdeckelement (1100) eine Vielzahl von aufeinanderfolgenden Schlaufen (1102) aus einem Streifen oder Band aus Material umfasst.

13. Herzklappenprothese nach Anspruch 12, wobei die Vielzahl von aufeinanderfolgenden Schlaufen (1102) des Streifens oder Bandes aus Material um die zwei verdünnten Strebenabschnitte (414), zwischen den Schultern des Spitzenbereichs (402, 404; 502), gewickelt sind.

14. Herzklappenprothese nach einem der Ansprüche 11 bis 13, weiter umfassend eine äußere Schürze (1150), die um eine Außenfläche des Rahmens (400; 500) angeordnet ist, und wobei sich das Abdeckelement (1100) durch die äußere Schürze (1150) hindurch erstreckt, so dass die äußere Schürze (1150) an dem Rahmen (400, 500) befestigt ist.

15. Herzklappenprothese nach einem der vorstehenden Ansprüche, weiter umfassend eine innere Schürze (1410), die an einer Innenfläche (1420) des Rahmens (400; 500) angeordnet und/oder mit dieser gekoppelt ist.

## Revendications

1. Valvule cardiaque prothétique comprenant :
un cadre annulaire radialement déployable et compressible (400 ; 500) comprenant :
une pluralité d'entretoises interconnectées définissant une pluralité de rangées de cellules agencées entre une extrémité d'entrée (408 ; 508) et une extrémité de sortie (406 ; 506) du cadre (400 ; 500), la pluralité d'entretoises interconnectées comprenant une pluralité d'entretoises de sortie (460 ; 560) définissant l'extrémité de sortie (406 ; 506) et une pluralité d'entretoises d'entrée (462 ; 562) définissant l'extrémité d'entrée (402, 404 ; 502), chaque entretoise de sortie (460 ; 560) comprenant deux parties d'entretoise inclinées (410 ; 510) interconnectées par une région de sommet (402, 404 ; 502), et chaque entretoise d'entrée (462 ; 562) comprenant deux parties d'entretoise inclinées (410 ; 510) interconnectées par une région de sommet (404 ; 502),
chaque région de sommet (402, 404 ; 502) s'incurvant entre une paire correspondante de deux parties d'entretoise inclinées (410 ; 510), chaque région de sommet (402, 404 ; 502) présentant une largeur rétrécie (416) par rapport aux parties d'entretoise inclinées, qui s'étend le long d'une longueur (425) de l'entretoise de sortie (460 ; 560) et de l'entretoise d'entrée (462 ; 562), et la largeur rétrécie (416) étant inférieure à une largeur (418) des deux parties d'entretoise inclinées (410 ; 510).

2. Valvule cardiaque prothétique selon la revendication 1, chaque région de sommet (402, 404 ; 502) formant un angle (440) entre les deux parties d'entretoise inclinées (410 ; 510) d'une entretoise de sortie correspondante (460 ; 560) et/ou d'une entretoise d'entrée (462 ; 562) correspondante qui est supérieur à 120 degrés et allant jusqu'à 140 degrés.

3. Valvule cardiaque prothétique selon la revendication 1 ou la revendication 2, chaque région de sommet (402, 404 ; 502) comportant une surface externe incurvée (428) d'un rayon de courbure qui est supérieur à 1 mm, la surface externe incurvée (428) s'étendant entre des surfaces externes des deux parties d'entretoise inclinées (410 ; 510) d'une entretoise de sortie correspondante (460 ; 560) et/ou d'une entretoise d'entrée correspondante (462 ; 562).

4. Valvule cardiaque prothétique selon l'une quelconque des revendications 1 à 3, la longueur de chaque région de sommet (402, 404 ; 502) étant située dans une plage de 0,9 mm à 2,2 mm.

5. Valvule cardiaque prothétique selon l'une quelconque des revendications 1 à 3, la longueur de chaque région de sommet (402, 404 ; 502) étant située dans une plage de 1,9 mm à 2,2 mm.

6. Valvule cardiaque prothétique selon l'une quelconque des revendications 1 à 3, la longueur de chaque région de sommet (402 ; 502) au niveau de l'extrémité de sortie (406 ; 506) étant située dans une plage de 1,8 mm à 2,4 mm et la longueur de chaque région de sommet (404 ; 502) au niveau de l'extrémité d'entrée (402 ; 502) étant située dans une plage de 0,8 mm à 1,2 mm.

7. Valvule cardiaque prothétique selon l'une quelconque des revendications 1 à 6, la largeur rétrécie (416) de chaque région de sommet (402, 404 ; 502) étant inférieure de 0,06 mm à 0,15 mm à la largeur (418) des deux parties d'entretoise inclinées (410 ; 510).

8. Valvule cardiaque prothétique selon l'une quelconque des revendications 1 à 7, comprenant en outre une pluralité de feuillets (204) fixés fermement au cadre (400 ; 500) et une pluralité de fenêtres de commissure (520) formées par des entretoises de la pluralité d'entretoises interconnectées formant des cellules d'une première rangée de cellules (324) de la pluralité de rangées de cellules, la première rangée de cellules (324) étant disposée au niveau de l'extrémité de sortie (406 ; 506) du cadre (400 ; 500) et chaque fenêtre de commissure (242, 520) étant conçue pour recevoir des languettes de commissure de deux feuillets adjacents (204) de la pluralité de feuillets (204).

9. Valvule cardiaque prothétique selon la revendication 8, chaque fenêtre de commissure (520) étant définie par des parties d'entretoise de fenêtre (514) s'étendant axialement qui forment une partie d'extrémité supérieure (516) au-dessus de la fenêtre de commissure et une partie d'extrémité inférieure (518) au-dessous de la fenêtre de commissure (520) et une longueur (528), dans une direction axiale par rapport à un axe longitudinal central du cadre (400 ; 500), de la partie d'extrémité supérieure (516) et de la partie d'extrémité inférieure (518) étant supérieure à la largeur (1630) des deux parties d'entretoise inclinées (410).

10. Valvule cardiaque prothétique selon la revendication 9, la partie d'extrémité supérieure (1616) comportant une région concave (1620) disposée en son sein, adjacente à une courbe convexe (1634) au niveau d'une base d'une première partie d'entretoise inclinée (1602a) des deux parties d'entretoise inclinées auxquelles la partie d'extrémité supérieure (1616) se relie et la courbe convexe (1636) s'étendant à partir d'une courbe concave (1634) dans la première partie d'entretoise inclinée (1602a).

11. Valvule cardiaque prothétique selon l'une quelconque des revendications 1 à 10, un élément de recouvrement (1100) étant enroulé autour de la région de sommet (402, 404 ; 502) du cadre (400 ; 500) et la recouvrant.

12. Valvule cardiaque prothétique selon la revendication 11, l'élément de recouvrement (1100) comprenant une pluralité de boucles consécutives (1102) d'une bande ou d'un ruban de matériau.

13. Valvule cardiaque prothétique selon la revendication 12, la pluralité de boucles consécutives (1102) de la bande ou du ruban de matériau étant enroulées autour des deux parties d'entretoise (414) amincies, entre les épaulements de la région de sommet (402, 404 ; 502).

14. Valvule cardiaque prothétique selon l'une quelconque des revendications 11 à 13, comprenant en outre une collerette externe (1150) disposée autour d'une surface externe du cadre (400 ; 500) et l'élément de recouvrement (1100) s'étendant à travers la collerette externe (1150) de telle sorte que la collerette externe (1150) est fixée fermement au cadre (400, 500).

15. Valvule cardiaque prothétique selon l'une quelconque des revendications précédentes, comprenant en outre une collerette interne (1410) agencée sur et/ou accouplée à une surface interne (1420) du cadre (400 ; 500).
